# EUROPEAN PATENT APPLICATION

(11) **EP 2 514 831 A1**
(43) Date of publication of application: **24.10.2012**
(21) Application number: 11162939.0
(22) Date of filing: 19.04.2011
(51) Int. Cl.: C12Q 1/26

(54) **New biomarker for measuring beta cell activity**

(71) Applicant: Pronota NV, 9052 Zwijnaarde (BE)
(72) Inventor: Kas, Koen, 2970, Schilde (BE); Vanpoucke, Griet, 9820, Merelbeke (BE); Moerman, Piet, 9831, Deurle (BE)
(74) Representative: Vanhalst, Koen

(57) **Abstract**

The application discloses Quiescin Q6 as a new biomarker for measuring beta cell activity due to different disorders; methods for predicting, diagnosing and/or prognosis of beta cell activity related disorders based on measuring the quantity or presence said biomarker; and kits and devices for use in measuring said biomarker and/or for performing said methods.

## Description

### FIELD OF THE INVENTION

The invention relates to protein- and/or peptide-based biomarkers and to agents specifically binding thereto, for use in predicting, diagnosing and/or prognosing diseases or conditions in subjects. More particularly, the application discloses a new biomarker for disorders linked to beta cell functioning or activity, preferably abnormal or reduced beta cell functioning, methods for measuring and monitoring said beta cell dysfunctioning in said disorders or conditions based on measuring said biomarker protein and/or peptides thereof; and kits and devices for measuring said protein and/or peptides thereof and/or performing said methods.

### BACKGROUND OF THE INVENTION

In many diseases and conditions, a favourable outcome of prophylactic and/or therapeutic treatments is strongly correlated with early and/or accurate prediction, diagnosis and/or prognosis of the disease or condition. Therefore, there exists a continuous need for additional and preferably improved manners for early and/or accurate prediction, diagnosis and/or prognosis of diseases and conditions to guide the treatment choices.

Type 1 and type 2 diabetes mellitus (T1D, T2D) have in common high blood glucose levels (hyperglycemia) that can cause serious health complications including ketoacidosis, kidney failure, heart disease, stroke, and blindness. While T2D is usually associated with obesity or older age and is mostly the result of insulin resistance, T1 D usually starts in people younger than 30 (also termed juvenile-onset diabetes), and is a chronic autoimmune disorder that precipitates in genetically susceptible individuals by environmental factors. The body's own immune system attacks the beta cells in the islets of Langerhans of the pancreas, destroying or damaging them sufficiently to reduce and eventually eliminate insulin production (For a review, cf. Van Belle et al., 2011, Physiol. Rev. 91:79-118). The problem with diagnosing beta cell damage, is that most methods actually require a tissue biopsy, which is highly invasive and hence undesirable. Many attempts have been made to visualise the pancreas using non-invasive methods. One example is the use of ferumoxtran, a long-circulating MNP (magnetic nanoparticle) that provides a signal when taken up by macrophages. It creates a signal in the pancreas reflecting the amount of nanoparticles that leak from pancreatic blood vessels into the surrounding pancreatic tissue, where the particles were taken up by macrophages. This reflects disruption of the pancreatic vasculature, specifically vessel leakage, associated with insulitis, and the signal correlates with the degree of islet inflammation. These results showed that there was significantly more nanoparticle accumulation in the pancreas of diabetes patients compared with in the pancreas of controls. That finding suggests vessels in the diseased pancreases were leakier and insulitis was present (cf. Gaglia, J.L. et al. J. Clin. Invest.; published online Dec. 1, 2010; doi:10.1172/JCI44339, and "Imaging islets" Martz, L. SciBX 3(48); doi:10.1038/scibx.2010.1433). Treatment of T1D is usually focussed on the regeneration of the beta cells population in the subject. Many studies using e.g. non-obese diabetic mice (NOD mice) have been used to follow the reduction in beta cell mass during the evolution of the disease. A complex interplay of initial (auto)inflammation, triggering proliferation of existing beta cells, generating more antigenic eptitopes prone for attack by auto-antibodies, finally result in a destruction of the beta cell population and T1D (cf. Akirav et al., 2008: Diabetes Vol.57:2883-2888). Following the beta cell activity thus is highly desirable in order to evaluate disease stage and decide on appropriate treatment thereof. In this respect, it is to be noted that the higher the residual beta cell mass in the subject is at the start of therapy, the better the results of said therapy will be. In addition, if beta cell grafting or transplantation is used as a treatment method, the ability to monitor the activity of the transplanted beta cells is also of great importance.

A change (increase or decrease) in beta cell activity is however not necessarily linked to T1D, which is an autoimmune disorder, but can be also be due to acute or chronic inflammation of the pancreas (pancreatitis). Alternatively, a change in beta cell activity can be due to pancreatic cancer or tumour formation (e.g. insulinoma), which can result in destruction of the beta cell population or can result in beta cell derived tumours or cancers.

Thus far, no non-invasive methods are available to evaluate the early changes in beta cell activity in a subject, i.e. before clinical manifestation of diabetes. The goal of the present invention is to overcome this problem by providing a new biomarker for beta cell activity that is detectable in a blood or serum sample of a subject.

### SUMMARY OF THE INVENTION

Having conducted extensive experiments and tests, the inventors identified Quiescin Q6 as a biomarker for evaluating beta cell activity, thereby providing a non-invasive tool for monitoring said activity in a subject.

In particular, using 1) blood samples of patients with aberrant beta cell activity due to e.g. pancreas cancer or pancreatitis, preferably chronic pancreatitis, and 2) blood samples from healthy subjects, the inventors realised that the quantity of the Quiescin Q6 protein in said samples displayed a behaviour which was predictive and/or indicative of aberrant or abnormal beta cell activity versus healthy subjects. In this respect, the Quiescin Q6 protein level detected in blood serum samples was both elevated in patients suffering from pancreatic cancer and chronic pancreatitis, pointing to a common physiological process in both disorders. Since it has been shown that beta cell activity is increased in pancreatitis and pancreas cancer, the inventors postulate that Quiescin Q6 is a marker suitable for monitoring beta cell activity in general, i.e. not linked to a specific disease or condition.

Hence, provided is the use of Quiescin Q6 as a biomarker for measuring and/or monitoring beta cell mass, beta cell quantity, and/or beta cell activity, or for monitoring insulin secretion or insulin synthesis by beta cells in a subject.

In a preferred embodiment, the beta cell activity in said subject is impaired or aberrant due to either one or more of the following conditions: pre-onset type 1 diabetes mellitus (T1D), pre-clinical T1D, early onset T1D, emerging T1D; pancreatitis such as: acute pancreatitis, alcohol induced acute pancreatitis, infectious acute pancreatitis, chronic pancreatitis, alcohol induced chronic pancreatitis, or biliar duct obstruction pancreatitis.

Alternatively, the beta cell activity in said subject is impaired or aberrant due to either one or more of the following conditions: insulin resistance, exhaustion of beta cells, emerging or developing T2D;

Furthermore, the beta cell activity in said subject can be impaired or aberrant due to pancreatic cancers or tumours.

The invention further provides a method for the measurement and/or monitoring of beta cell mass and/or activity in a subject, comprising measuring the quantity of Quiescin Q6 in a blood sample from the subject.

In a preferred embodiment, said method comprises the steps of:
(i) measuring the quantity of Quiescin Q6 in a blood sample from the subject;
(ii) comparing the quantity of said Quiescin Q6 measured in (i) with a reference value of the quantity of said Quiescin Q6, said reference value representing the beta cell activity in a reference sample;
(iii) finding a deviation or no deviation of the quantity of the Quiescin Q6 measured in (i) from the reference value; and
(iv) attributing said finding of deviation or no deviation to a particular diagnosis, prediction and/or prognosis of aberrant beta cell activity in the subject.

In a further preferred embodiment, the method according to the present invention is used for determining whether a subject is or is not (such as, still is, or is no longer), or is to the same extent, in need of a therapeutic or prophylactic treatment of a disease or disorder selected from the group consisting of: pre-onset type 1 diabetes mellitus (T1D), pre-clinical T1D, early onset T1D, emerging T1D; pancreatitis such as: acute pancreatitis, alcohol induced acute pancreatitis, infectious acute pancreatitis, chronic pancreatitis, alcohol induced chronic pancreatitis, or biliar duct obstruction pancreatitis.

In an alternative embodiment, the method according to the present invention is used for determining whether a subject is or is not (such as, still is, or is no longer), or is to the same extent, in need of a therapeutic or prophylactic treatment of a disease or disorder selected from the group consisting of: insulin resistance, exhaustion of beta cells, emerging or developing T2D.

In a further embodiment, the method according to the present invention is used for determining whether a subject is or is not (such as, still is, or is no longer), or is to the same extent, in need of a therapeutic or prophylactic treatment of pancreatic cancer or tumours.

In a preferred embodiment, the subject does not yet suffer from clinically manifest signs or symptoms of the condition linked to impaired or aberrant beta cell activity and the probability, chance or risk that the subject will develop said disease or disorder is predicted.

In certain embodiments, the method according to the invention can be performed at two or more successive time points, and attributing a finding of deviation or no deviation in Quiescin Q6 levels between said time points to a change in the beta cell activity and/or in disease prediction, staging, monitoring or progression.

In an alternative embodiment, the method according to the present invention further comprises measuring the presence or absence and/or quantity of one or more other biomarkers or parameters useful for the measurement of beta cell mass or activity, such as insulin, or pro-insulin production or secretion, blood glucose level, VMAT2 expression, etc..

In a particularly preferred embodiment of the method according to the invention, the quantity of Quiescin Q6 is measured using (a) binding agent(s) capable of specifically binding to Quiescin Q6 and/or to fragments thereof, or using an immunoassay technology, using a mass spectrometry analysis method, using a chromatography method, or using a combination of any of said methods.

The invention also provides for the use of a kit comprising:
(i) means for measuring the quantity of Quiescin Q6 in a sample from the subject, and optionally and preferably
(ii) a reference value of the quantity of Quiescin Q6 or means for establishing said reference value,
wherein said reference value represents the beta cell activity of a known diagnosis, prediction and/or prognosis of a condition linked to impaired or aberrant beta cell activity, or represents a healthy subject,
for measuring beta cell activity in a blood sample of a subject.

Preferably, said means for measuring the quantity of Quiescin Q6 in a sample is a binding agent capable of specifically binding to said marker, such as those selected from the group consisting of: antibodies or fragments thereof, aptamers, photoaptamers, proteins, peptides, polypeptides, peptidomimetics, DARPINS, or small molecules,

The invention further provides for the use of a Quiescin Q6 protein, polypeptide, or peptide and/or a fragment thereof, preferably in a known quantity or concentration, for diagnosing a beta cell related disorder in a subject, by detecting the presence of auto-antibodies to Quiescin Q6 in a sample of the subject.

In a further aspect, the invention provides for the use of a testing device capable of measuring the quantity of Quiescin Q6 and/or fragments thereof in a sample from a subject comprising:
(i) optionally means for obtaining a sample from the subject,
(ii) means for measuring the quantity of Quiescin Q6 and/or fragments in said sample, and
(iii) means for visualising the quantity of Quiescin Q6 and/or fragments measured in the sample, for measuring beta cell activity in a sample of a subject.

In a preferred embodiment of the invention, the method as defined herein is used for the selection of patients that are at risk of progressing towards beta cell destruction, dysfunction, or abnormal beta cell activity or turnover, based on the levels of Quiescin Q6 in a blood sample of the subject. In a further preferred embodiment of the invention, the method as defined herein is used for deciding on the timing of therapy to prevent beta cell destruction, dysfunction, or abnormal beta cell activity or turnover, based on the levels of Quiescin Q6 in a blood sample of the subject.

In yet another embodiment of the invention, the method as defined herein is used for deciding on the type of therapy to prevent beta cell destruction/dysfunction or abnormal beta cell activity or turnover, based on the levels of Quiescin Q6 in a blood sample of the subject.

In an alternative embodiment of the invention, the method as defined herein is used for designing or monitoring immunomodulating therapies, wherein the monitoring of the beta cell activity is used for adapting the treatment regimen or for selecting the most appropriate drugs or monoclonal antibodies for said immunomodulatory treatment.

In a preferred embodiment of the invention, the method as defined herein is used for beta cell transplantation surveillance, wherein said method is used to measure the beta cell activity of the subject at certain time points after beta cell transplantation and wherein a change of the beta cell activity level indicates a change in the activity of the grafted beta cells and hence gives a good reflection of the transplantation success and evolution.

In a further preferred embodiment of the invention, the method as defined herein is used for monitoring immuno-suppression treatment, wherein increased immuno-suppression therapy is advisable in case of reduction in beta cell activity, or wherein maintained or decreased immuno-suppression therapy is advisable in case the beta cells activity is improving or in a steady state as compared to the pre-transplantation level.

The method according to the invention can also be used for detecting or predicting the onset, or quantifying the degree of insulin resistance in a subject.

In any one of the embodiments of the methods as defined herein, said beta cell mass or activity reflects either one of: the capacity of insulin synthesis or secretion, the amount of insulin synthesis or secretion over a certain period, or the maximal amount of insulin secretion.

In any one of the embodiments of the methods as defined herein, said beta cell mass or activity reflects either one of: the number or volume of beta cells, the number or volume of active beta cells, the number or volume of dormant beta cells, the rate of beta cell destruction, proliferation, regeneration or granulation of the beta cell population or tissue.

In any one of the embodiments of the methods as defined herein, the severity of said impaired beta cell functioning is measured in terms of either one of: the degree of autoimmune destruction of beta cells, the severity of Langerhans infiltration with white blood cells, the severity of auto-antigenecity, the correlation with the amount, titer or presence of auto-antibodies related to T1D, such as GAD-65, pro-insulin, IA2, etc..

Hence, an elevated quantity or a reduced quantity (i.e., a deviation) of Quiescin Q6 in a sample from a subject compared to a reference value representing the beta cell activity of a healthy or non-diseased subject with reference to any one of the conditions as taught herein (i.e., healthy state) or representing a good prognosis for a diseases or conditions as taught herein, may indicate respectively that the subject has or is at risk of having the diseases or conditions as taught herein or indicates a poor prognosis for the diseases or conditions as taught herein in the subject.

By means of example only and without any limitation, (a) an elevated quantity (i.e., a deviation) of Quiescin Q6 in a sample from a subject compared to a reference value representing the prediction or diagnosis of no diseases or conditions as taught herein (i.e., healthy state) or representing a good prognosis for a diseases or conditions as taught herein may indicate respectively that the subject has increased beta cell activity and/or is at risk of having a disease or conditions related to increased beta cell activity; while (b) a reduced quantity (i.e., a deviation) of Quiescin Q6 in a sample from a subject compared to a reference value representing the prediction or diagnosis of no diseases or conditions as taught herein (i.e., healthy state) or representing a good prognosis for a diseases or conditions as taught herein may indicate respectively that the subject has decreased beta cell activity and/or is at risk of having a disease or conditions related to decreased beta cell activity.

The present methods for the diagnosis, prediction, prognosis and/or monitoring of a diseases or conditions as taught herein may also assess additional biomarkers or parameters such as: insulin or pro-insulin production or secretion, blood glucose level, (Vesicular monoamine transporter 2) VMAT2 expression etc., in a subject. Each so-measured biomarker or parameter may be evaluated separately and independently, or one may generate a biomarker profile from the quantities of said two or more biomarkers or parameters.

Accordingly, disclosed is also a method for the diagnosis, prediction and/or prognosis of diseases or conditions as taught herein in a subject comprising the steps: (i) measuring the quantity of Quiescin Q6 and one or more other biomarkers or parameters for diseases or conditions as taught herein in the sample, preferably a blood or serum sample, from the subject; (ii) using the measurements of (i) to establish a subject profile of the quantity of the two or more markers or parameters; (iii) comparing said subject profile of (ii) to a reference profile of the quantity of said two or more markers or parameters, said reference profile representing a known diagnosis, prediction and/or prognosis of the diseases or conditions as taught herein; (iv) finding a deviation or no deviation of the subject profile of (ii) from the reference profile; (v) attributing said finding of deviation or no deviation to a particular diagnosis, prediction and/or prognosis of the diseases or conditions as taught herein .

Applying the method at two or more successive time points allows for monitoring the diseases or conditions as taught herein.

In an embodiment, a method for monitoring diseases or conditions as taught herein (or for monitoring the probability of developing diseases or conditions as taught herein) comprises the steps of: (i) measuring the quantity of Quiescin Q6 in samples, preferably blood or serum samples, from a subject from two or more successive time points; (ii) comparing the quantity of Quiescin Q6 between the samples as measured in (i); (iii) finding a deviation or no deviation of the quantity of Quiescin Q6 between the samples as compared in (ii); and (iv) attributing said finding of deviation or no deviation to a change in the diseases or conditions as taught herein (or to a change in the probability of developing diseases or conditions as taught herein) in the subject between the two or more successive time points. The method thus allows monitoring the diseases or conditions as taught herein or the risk of developing diseases or conditions as taught herein in a subject over time.

In another embodiment, a method for monitoring diseases or conditions as taught herein (or for monitoring the probability of developing diseases or conditions as taught herein) comprises the steps of: (i) measuring the quantity of Quiescin Q6 and one or more other biomarkers for diseases or conditions as taught herein in samples, preferably blood or serum samples, from a subject from two or more successive time points; (ii) using the measurements of (i) to establish subject profiles of the quantity of the two or more markers at the two or more successive time points; (iii) comparing the subject profiles as established in (ii); (iv) finding a deviation or no deviation between the subject profiles as compared in (iii); and (v) attributing said finding of deviation or no deviation to a change in the diseases or conditions as taught herein (or to a change in the probability of developing diseases or conditions as taught herein) in the subject between the two or more successive time points. The method thus allows monitoring diseases or conditions as taught herein or the risk of developing diseases or conditions as taught herein in a subject over time.

Without limitation, such successive time points may be days apart, weeks apart, about 2 weeks or more apart, preferably about 4 weeks or more apart, e.g., about 6 or 8 weeks apart, or also preferably about 10 weeks or more apart, e.g., about 12 weeks or 15 weeks apart.

Throughout the present disclosure, methods for monitoring any one of the diseases or conditions as taught herein can inter alia allow to predict the occurrence (pre-clinical, pre-onset, onset, or emerging stage) of the disease or condition, or to monitor the progression, aggravation, alleviation or recurrence of the disease or condition, or response to treatment or to other external or internal factors, situations or stressors, etc.

Advantageously, monitoring methods as taught herein may be applied in the course of a medical treatment of the subject, preferably medical treatment aimed at alleviating the so-monitored disease or condition. Such monitoring may be comprised, e.g., in decision making whether a patient may be discharged, needs a change in treatment or needs further hospitalisation. As intended herein, a reference to monitoring of a disease or condition also specifically includes monitoring of the probability, risk or chance of a subject to develop the disease or condition, i.e., monitoring change(s) in said probability, risk or chance over time.

Similarly, throughout the present disclosure, methods for the prediction or prognosis of any one of the diseases or conditions as taught herein can inter alia allow to predict or make a prognosis of the occurrence of the disease or condition, or to predict or make a prognosis of the progression, aggravation, alleviation or recurrence of the disease or condition or response to treatment or to other external or internal factors, situations or stressors, etc.

The inventors further realised that the evaluation of the Quiescin Q6 biomarker as taught herein at successive time points during treatment or observation of the subject may also allow for the diagnosis, prediction and/or prognosis of diseases or conditions as taught herein. For example, where the difference between the quantities of said marker at said successive time points deviates from the difference between the quantities of said marker measured at corresponding time points in subjects who would not develop diseases or conditions as taught herein, such deviation may indicate that the subject has or is at risk of developing diseases or conditions as taught herein.

Accordingly, provided is also a method for the diagnosis, prediction and/or prognosis of diseases or conditions as taught herein in a subject comprising the steps of: (i) measuring the quantity of Quiescin Q6 in a sample, preferably a blood or serum sample, from the subject from a first time point; (ii) measuring the quantity of Quiescin Q6 in a sample, preferably a blood or serum sample, from the subject from a successive second time point; (iii) calculating the difference between the quantities of Quiescin Q6 as measured in (i) and (ii); (iv) comparing the difference as calculated in (iii) with a reference value of the difference between the quantity Quiescin Q6 at said first and second time points, said reference value representing a known diagnosis, prediction and/or prognosis of the diseases or conditions as taught herein; (v) finding a deviation or no deviation of the difference as calculated in (iii) from the reference value; and (iv) attributing said finding of deviation or no deviation to a particular diagnosis, prediction and/or prognosis of diseases or conditions as taught herein in the subject.

Also disclosed is a method for the diagnosis, prediction and/or prognosis of diseases or conditions as taught herein in a subject comprising the steps of: (i) measuring the quantity of Quiescin Q6 and one or more other biomarkers for diseases or conditions as taught herein in a sample, preferably a blood or serum sample, from the subject from a first time point; (ii) using the measurements of (i) to establish a subject profile of the quantity of the two or more markers at said first time point; (iii) measuring the quantity of said two or more markers in a sample from the subject from a successive second time point; (iv) using the measurements of (iii) to establish a subject profile of the quantity of the two or more markers at said second time point; (v) calculating the difference between the subject profiles as established in (ii) and (iv); (vi) comparing the difference as calculated in (v) with a reference profile of the difference between the quantity of said two or more markers at said first and second time points, said reference profile representing a known diagnosis, prediction and/or prognosis of diseases or conditions as taught herein; (vii) finding a deviation or no deviation of the difference as calculated in (v) from the reference profile; and (viii) attributing said finding of deviation or no deviation to a particular diagnosis, prediction and/or prognosis of diseases or conditions as taught herein in the subject.

For example, where the difference (DS) calculated between the quantities of a given marker measured in a subject at said first and second time points deviates from the corresponding difference (DN) as observed in normal healthy subjects, the deviation may indicate that the subject has or is at risk of having diseases or conditions as taught herein. Without limitation, a deviation may be pronounced where DS > DN or where DS < DN or where DS > 0 whereas DN < 0, or where DS < 0 whereas DN > 0. The difference may be suitable expressed as an arithmetic operation, such as, e.g., subtraction or division (e.g., slope, ratio).

Without limitation, such successive time points may be about 2 weeks or more apart, preferably about 4 weeks or more apart, e.g., about 6 or 8 weeks apart, or also preferably about 10 weeks or more apart, e.g., about 12 weeks or 15 weeks apart.

Also disclosed is a method to determine whether a subject is or is not (such as, e.g., still is, or is no longer), or is to the same extent, in need of a therapeutic or prophylactic (preventative) treatment of diseases or conditions as taught herein, comprising: (i) measuring the quantity of Quiescin Q6 in the sample, preferably a blood or serum sample, from the subject; (ii) comparing the quantity of the Quiescin Q6 measured in (i) with a reference value of the quantity of Quiescin Q6, said reference value representing a known diagnosis, prediction and/or prognosis of the diseases or conditions as taught herein; (iii) finding a deviation or no deviation of the quantity of the Quiescin Q6 measured in (i) from said reference value; (iv) inferring from said finding the presence or absence of a need for a therapeutic or prophylactic treatment of the diseases or conditions as taught herein.

Also disclosed is a method to determine whether a subject is or is not (such as, e.g., still is, or is no longer) in need of a therapeutic or prophylactic (preventative) treatment of diseases or conditions as taught herein , comprising: (i) measuring the quantity of Quiescin Q6 and one or more other biomarkers for diseases or conditions as taught herein in the sample, preferably a blood or serum sample, from the subject; (ii) using the measurements of (i) to establish a subject profile of the quantity of the two or more markers; (iii) comparing said subject profile of (ii) to a reference profile of the quantity of said two or more markers, said reference profile representing a known diagnosis, prediction and/or prognosis of the diseases or conditions as taught herein; (iv) finding a deviation or no deviation of the subject profile of (ii) from the reference profile; (v) inferring from said finding the presence or absence of a need for a therapeutic or prophylactic treatment of the diseases or conditions as taught herein.

A treatment may be particularly indicated where the method allows for a conclusion that the subject has or is at risk of having the diseases or conditions as taught herein or has a poor prognosis for the diseases or conditions as taught herein. Without limitation, a patient having diseases or conditions as taught herein upon admission to or during stay in a medical care centre may be tested as taught herein for the necessity of continuing the treatment of said diseases or conditions as taught herein, and may be discharged when such treatment is no longer needed or is needed only to a given limited extent. Exemplary therapeutic and prophylactic treatments of diseases or conditions as taught herein encompass without limitation the administration of antiinflammatory agents or can encompass means for changing (reducing or increasing) the protein level of Quiescin Q6 or changing (reducing or increasing)the mRNA level encoding Quiescin Q6 in the subject.

In addition, the Quiescin Q6 biomarker can be used as a surrogate marker or comparative diagnostic for developing and designing therapies intended to increase beta cell activity, insulin synthesis and/or insulin secretion.

In this respect, one can envisage a screening method or model system, wherein Quiescin Q6 levels are measured to evaluate the effect of a candidate drug or agent intended to increase beta cell activity, insulin synthesis and/or insulin secretion. The difference in Quiescin Q6 levels in the presence versus the absence of the candidate agent represents the effect of said candidate agent on the Quiescin Q6 level and hence also on its ability to modulate beta cell activity. The uses and methods involving evaluation of Quiescin Q6 levels as taught herein may principally be carried out for any mammalian subject. Preferably said subject is human.

The uses and methods involving evaluation of Quiescin Q6 as taught herein may be preferably intended and employed for the early prediction of diseases or conditions as taught herein in subjects, particularly in subjects not yet having clinically manifest (i.e., active) diseases or conditions as taught herein. Such prediction may preferably indicate a probability, chance or risk that a tested subject will develop clinically manifest diseases or conditions as taught herein, for example within a certain time period.

Any one prediction, diagnosis, prognosis and/or monitoring use or method as taught herein may preferably allow for sensitivity and/or specificity (preferably, sensitivity and specificity) of at least 50%, at least 60%, at least 70% or at least 80%, e.g., ≥ 85% or ≥ 90% or ≥95%, e.g., between about 80% and 100% or between about 85% and 95%.

Throughout the present disclosure, methods for monitoring any one disease or condition as taught herein can inter alia allow to predict the occurrence of the disease or condition, or to monitor the progression, aggravation, alleviation or recurrence of the disease or condition, or response to treatment or to other external or internal factors, situations or stressors, etc. Advantageously, monitoring methods as taught herein may be applied in the course of a medical treatment of the subject, preferably medical treatment aimed at alleviating the so-monitored disease or condition. Such monitoring may be comprised, e.g., in decision making whether a patient may be discharged, needs a change in treatment or needs further hospitalisation. As intended herein, a reference to monitoring of a disease or condition also specifically includes monitoring of the probability, risk or chance of a subject to develop the disease or condition, i.e., monitoring change(s) in said probability, risk or chance over time.

Similarly, throughout the present disclosure, methods for the prediction or prognosis of any one disease or condition as taught herein can inter alia allow to predict or make a prognosis of the occurrence of the disease or condition, or to predict or make a prognosis of the progression, aggravation, alleviation or recurrence of the disease or condition or response to treatment or to other external or internal factors, situations or stressors, etc.

As used throughout this specification, measuring the levels of any one or more biomarker(s) in a sample from a subject may particularly denote that the examination phase of a method comprises measuring the quantity of said one or more biomarker(s) in the sample from the subject. One understands that methods for the diagnosis, prediction, prognosis and/or monitoring of diseases and conditions as taught herein generally comprise an examination phase in which data is collected from and/or about the subject.

The uses and methods for the diagnosis, prediction, prognosis and/or monitoring of the diseases and conditions taught herein may be used in subjects who have not yet been diagnosed as having such (for example, preventative screening), or who have been diagnosed as having such, or who are suspected of having such (for example, display one or more characteristic signs and/or symptoms), or who are at risk of developing such (for example, genetic predisposition; presence of one or more developmental, environmental or behavioural risk factors). The uses and methods may also be used to detect various stages of progression or severity of the diseases and conditions. The uses and methods may also be used to detect response of the diseases and conditions to prophylactic or therapeutic treatments or other interventions. The uses and methods can furthermore be used to help the medical practitioner in deciding upon worsening, status-quo, partial recovery, or complete recovery of the subject from the diseases and conditions, resulting in either further treatment or observation or in discharge of the patient from a medical care centre.

Also, the uses and methods for the diagnosis, prediction, prognosis and/or monitoring of the diseases and conditions taught herein may be employed for population screening, such as, e.g., screening in a general population or in a population stratified based on one or more criteria, e.g., age, ancestry, occupation, presence or absence of risk factors of the respective diseases and conditions, etc.

In the uses and methods for the prediction, diagnosis, prognosis and/or monitoring of diseases and disorders linked to impaired beta cell activity, such as those selected from the group consisting of: T1D, pancreatitis, or pancreatic cancer or tumour formation, the measurement of Quiescin Q6 may be combined with the assessment of one or more further biomarkers or clinical parameters relevant for the respective diseases and conditions.

Hence, also provided are methods for the diagnosis, prediction, prognosis and/or monitoring of impaired or aberrant beta cell activity, caused by conditions such as those selected from the group consisting of: pre-onset type 1 diabetes mellitus (T1D); pre-clinical T1D, early onset T1D, emerging T1D; pancreatitis such as: acute pancreatitis, alcohol induced acute pancreatitis, infectious acute pancreatitis, chronic pancreatitis, alcohol induced chronic pancreatitis, or biliar duct obstruction pancreatitis in a subject as taught above, further comprising measuring the presence or absence and/or level of one or more such other markers in the sample from the subject. Specifically provided are such methods wherein the examination phase of the methods further comprises measuring the presence or absence and/or quantity of one or more such other markers in the sample from the subject. Any known or yet unknown suitable markers can be used. Hence, also provided are methods for the diagnosis, prediction, prognosis and/or monitoring of impaired or aberrant beta cell activity, caused by conditions such as those selected from the group consisting of: a) insulin resistance, exhaustion of beta cells, emerging or developing T2D; or b) pancreatic cancer or tumour formation; in a subject as taught above, further comprising measuring the presence or absence and/or level of one or more such other markers in the sample from the subject. Specifically provided are such methods wherein the examination phase of the methods further comprises measuring the presence or absence and/or quantity of one or more such other markers in the sample from the subject. Any known or yet unknown suitable markers can be used.

A reference throughout this specification to "other (bio)markers" generally encompasses such other markers which are useful for the diagnosis, prediction, prognosis and/or monitoring of the diseases and conditions as disclosed herein. By means of example and not limitation, biomarkers useful in evaluating beta cell function and insulin resistance include for example VMAT2, which is an indicator of beta cell mass, C-peptide, insulin synthesis or secretion, or blood glucose level, free fatty acid level (FFA's), and magnetic nanoparticle effusion technique (cf. Martz, L. *SciBX* 3(48); doi:10.1038/scibx.2010.1433)can also be used to evaluate residual beta cell activity in a T1 D, or T2D subject.

One shall appreciate that the presence or absence and/or quantity of such other biomarkers may be evaluated each separately and independently, or the presence or absence and/or quantity of such other biomarkers may be included within subject profiles or reference profiles established in the methods disclosed herein.

Reference values as employed herein may be established according to known procedures previously employed for other biomarkers. Such reference values may be established either within (i.e., constituting a step of) or external to (i.e., not constituting a step of) the methods as taught herein. Accordingly, any one of the methods taught herein may comprise a step of establishing a reference value for the quantity of one or more markers as taught herein, said reference value representing either (a) a prediction or diagnosis of the absence of the diseases or conditions as taught herein or a good prognosis thereof, or (b) a prediction or diagnosis of the diseases or conditions as taught herein or a poor prognosis thereof.

A further aspect provides a method for establishing a reference value for the quantity of Quiescin Q6 said reference value representing:(a) a prediction or diagnosis of the absence of impaired or aberrant beta cell activity due to any one of the conditions as taught herein, or a good prognosis thereof, or
(b) a prediction or diagnosis of impaired or aberrant beta cell activity due to any one of the conditions as taught herein, or a poor prognosis thereof,
comprising:
(i) measuring the quantity of Quiescin Q6 in:
   (i a) one or more samples, preferably blood or serum samples, from one or more subjects not having the respective diseases or conditions or not being at risk of having such or having a good prognosis for such, or
   (i b) one or more samples , preferably blood or serum samples from one or more subjects having the respective diseases or conditions or being at risk of having such or having a poor prognosis for such, and
(ii) storing the quantity of Quiescin Q6:
   (ii a) as measured in (i a) as the reference value representing the prediction or diagnosis of the absence of the respective diseases or conditions or representing the good prognosis therefore, or
   (ii b) as measured in (i b) as the reference value representing the prediction or diagnosis of the respective diseases or conditions or representing the poor prognosis therefore.

The present methods may otherwise employ reference profiles for the quantity of Quiescin Q6, and optionally the presence or absence and/or quantity of one or more other biomarkers or parameters as disclosed herein, which may be established according to known procedures previously employed for other biomarkers. Such reference profiles may be established either within (i.e., constituting a step of) or external to (i.e., not constituting a step of) the present methods. Accordingly, the methods taught herein may comprise a step of establishing a reference profile for the quantity of any one, any two or more markers as taught herein and optionally the presence or absence and/or quantity of one or more other biomarkers, said reference profile representing either (a) a prediction or diagnosis of the absence of the diseases or conditions as taught herein or a good prognosis therefore, or (b) a prediction or diagnosis of the diseases or conditions as taught herein or a poor prognosis therefore.

A further aspect provides a method for establishing a reference profile for the quantity of Quiescin Q6 and optionally the presence or absence and/or quantity of one or more other biomarkers useful for the diagnosis, prediction, prognosis and/or monitoring of diseases and disorders linked to impaired beta cell activity, such as those selected from the group consisting of: pre-onset type 1 diabetes mellitus (T1 D), pre-clinical T1 D, early onset T1 D, and emerging T1 D, pancreatitis such as acute pancreatitis, alcohol induced acute pancreatitis, infectious acute pancreatitis, chronic pancreatitis, alcohol induced chronic pancreatitis, or biliar duct obstruction pancreatitis, pancreatitis, said reference profile representing:
(a) a prediction or diagnosis of the absence of the respective diseases or conditions or a good prognosis therefore, or
(b) a prediction or diagnosis of the respective diseases or conditions or a poor prognosis therefore,
   comprising:
   (i) measuring the quantity of said one, two or more markers as taught herein and the presence or absence and/or quantity of said one or more other biomarkers in:
      (i a) one or more samples, preferably blood or serum samples from one or more subjects not having the respective diseases or conditions or not being at risk of having such or having a good prognosis for such; or
      (i b) one or more samples, preferably blood or serum samples from one or more subjects having the respective diseases or conditions or being at risk of having such or having a poor prognosis for such;
(ii)
   (ii a) using the measurements of (i a) to create a profile of the quantity of said one, two or more markers as taught herein and the presence or absence and/or quantity of said one or more other biomarkers; or
   (ii b) using the measurements of (i b) to create a profile of the quantity of said one, two or more markers as taught herein and the presence or absence and/or quantity of said one or more other biomarkers;
(iii)
   (iii a) storing the profile of (ii a) as the reference profile representing the prediction or diagnosis of the absence of the respective diseases or conditions or representing the good prognosis therefore; or
   (iii b) storing the profile of (ii b) as the reference profile representing the prediction or diagnosis of the respective diseases conditions or representing the poor prognosis therefore.

Alternatively, in said method for establishing a reference profile, said conditions are selected from the group consisting of: insulin resistance, exhaustion of beta cells, emerging or developing T2D.

Furthermore, in said method for establishing a reference profile, said conditions linked to beta cell activity can be pancreatic cancer or tumour formation.

Further provided is a method for establishing a base-line or reference value in a subject, comprising: (i) measuring the quantity of Quiescin Q6 in the sample, preferably a blood or serum sample, from the subject at different time points wherein the subject is not suffering from a condition linked to impaired or aberrant beta cell activity selected from the group consisting of: pre-onset type 1 diabetes mellitus (T1D), pre-clinical T1D, early onset T1D, emerging T1D: pancreatitis such as acute pancreatitis, alcohol induced acute pancreatitis, infectious acute pancreatitis, chronic pancreatitis, alcohol induced chronic pancreatitis, or biliar duct obstruction pancreatitis, and (ii) calculating the range or mean value of the subject, which is the base-line or reference value for said subject. The quantity of Quiescin Q6 and/or the presence or absence and/or quantity of the one or more other biomarkers may be measured by any suitable technique such as may be known in the art.

Alternatively, in said method for establishing a base-line or reference value in a subject, said disorder can be selected from the group consisting of: insulin resistance, exhaustion of beta cells, emerging or developing T2D.

Furthermore, in said method for establishing a base-line or reference value in a subject, said disorder can be pancreatic cancer or tumour formation.

For example, one may employ binding agents capable of specifically binding to the respective biomarkers and/or to fragments thereof. Binding agent may be inter alia an antibody, aptamer, photoaptamer, protein, peptide, peptidomimetic or a small molecule. For instance, one may employ an immunoassay technology or a mass spectrometry analysis method or a chromatography method, or a combination of said methods.

Further disclosed is a kit for the diagnosis, prediction, prognosis and/or monitoring of impaired or aberrant beta cell activity due to a condition selected from the group consisting of: pre-onset type 1 diabetes mellitus (T1 D), pre-clinical T1 D, early onset T1 D, emerging T1 D; pancreatitis such as: acute pancreatitis, alcohol induced acute pancreatitis, infectious acute pancreatitis, chronic pancreatitis, alcohol induced chronic pancreatitis, or biliar duct obstruction pancreatitis, in a subject, the kit comprising (i) means for measuring the quantity of Quiescin Q6 in a sample, preferably a blood or serum sample, from the subject, and optionally and preferably (ii) a reference value of the quantity of Quiescin Q6 or means for establishing said reference value, wherein said reference value represents a known diagnosis, prediction and/or prognosis of the respective diseases or conditions, or represents a healthy subject. The kit thus allows one to: measure the quantity of Quiescin Q6 in the sample, preferably a blood or serum sample, from the subject by means (i); compare the quantity of Quiescin Q6 measured by means (i) with the reference value of (ii) or established by means (ii); find a deviation or no deviation of the quantity of Quiescin Q6 measured by means (i) from the reference value of (ii); and consequently attribute said finding of deviation or no deviation to a particular diagnosis, prediction and/or prognosis of the respective diseases or conditions in the subject.

A further embodiment provides a kit for the diagnosis, prediction, prognosis and/or monitoring of impaired or aberrant beta cell activity due to a condition selected from the group consisting of: pre-onset type 1 diabetes mellitus (T1D), pre-clinical T1D, early onset T1D, emerging T1D; pancreatitis such as: acute pancreatitis, alcohol induced acute pancreatitis, infectious acute pancreatitis, chronic pancreatitis, alcohol induced chronic pancreatitis, or biliar duct obstruction pancreatitis, in a subject, the kit comprising (i) means for measuring Quiescin Q6 in a sample, preferably a blood or serum sample, from the subject and (ii) optionally, means for measuring the presence or absence and/or quantity of Quiescin Q6 in the sample from the subject, and optionally and preferably (iii) means for establishing a subject profile of the quantity of Quiescin Q6 as taught herein and optionally the presence or absence and/or quantity of Quiescin Q6, and optionally and preferably (iv) a reference profile of the quantity of Quiescin Q6 as taught herein and optionally the presence or absence and/or quantity of Quiescin Q6, or means for establishing said reference profile, said reference profile representing a known diagnosis, prediction and/or prognosis of the respective diseases or conditions. Such kit thus allows one to: measure the quantity of Quiescin Q6 and optionally the presence or absence and/or quantity of Quiescin Q6 in the sample from the subject by respectively means (i) and (ii); establish (e.g., using means included in the kit or using suitable external means) a subject profile of the quantity of Quiescin Q6 and the presence or absence and/or quantity of Quiescin Q6 based on said measurements; compare the subject profile with the reference profile of (iv) or established by means (iv); find a deviation or no deviation of said subject profile from said reference profile; and consequently attribute said finding of deviation or no deviation to a particular diagnosis, prediction and/or prognosis of the respective diseases or conditions in the subject.

The means for measuring the quantity of Quiescin Q6 and/or the presence or absence and/or quantity of Quiescin Q6 in the present kits may comprise, respectively, one or more binding agents capable of specifically binding to Quiescin Q6 and/or to fragments thereof. Binding agent may be inter alia an antibody, aptamer, photoaptamer, protein, peptide, peptidomimetic or a small molecule. A binding agent may be advantageously immobilised on a solid phase or support. The present kits may employ an immunoassay technology or mass spectrometry analysis technology or chromatography technology, or a combination of said technologies.

Disclosed is thus also a kit for the diagnosis, prediction, prognosis and/or monitoring of impaired or aberrant beta cell activity due to a condition selected from the group consisting of: pre-onset type 1 diabetes mellitus (T1 D), pre-clinical T1 D, early onset T1 D, emerging T1 D; pancreatitis such as: acute pancreatitis, alcohol induced acute pancreatitis, infectious acute pancreatitis, chronic pancreatitis, alcohol induced chronic pancreatitis, or biliar duct obstruction pancreatitis, comprising: (a) one or more binding agents capable of specifically binding to Quiescin Q6 and/or to fragments thereof; (b) preferably, a known quantity or concentration of Quiescin Q6 and/or a fragment thereof (e.g., for use as controls, standards and/or calibrators); (c) preferably, a reference value of the quantity of Quiescin Q6, or means for establishing said reference value. Said components under (a) and/or (c) may be suitably labelled as taught elsewhere in this specification.

Also disclosed is a kit for the diagnosis, prediction and/or prognosis of impaired or aberrant beta cell activity due to a condition selected from the group consisting of: pre-onset type 1 diabetes mellitus (T1D), pre-clinical T1D, early onset T1D, emerging T1D; pancreatitis such as acute pancreatitis, alcohol induced acute pancreatitis, infectious acute pancreatitis, chronic pancreatitis, alcohol induced chronic pancreatitis, or biliar duct obstruction pancreatitis, comprising: (a) one or more binding agents capable of specifically binding to Quiescin Q6 and/or to fragments thereof; (b) optionally one or more binding agents capable of specifically binding to one or more other biomarkers or parameters as defined herein; (c) preferably, a known quantity or concentration of Quiescin Q6 and/or a fragment thereof and optionally a known quantity or concentration of said one or more other biomarkers (e.g., for use as controls, standards and/or calibrators); (d) preferably, a reference profile of the quantity of Quiescin Q6 and optionally of the presence or absence and/or quantity of said one or more other biomarkers, or means for establishing said reference profiles. Said components under (a), (b) and/or (c) may be suitably labelled as taught elsewhere in this specification.

Further disclosed is the use of the kit as described herein for the diagnosis, prediction, prognosis and/or monitoring impaired or aberrant beta cell activity due to a condition selected from the group consisting of: pre-onset type 1 diabetes mellitus (T1D), pre-clinical T1D, early onset T1D, emerging T1D; pancreatitis such as acute pancreatitis, alcohol induced acute pancreatitis, infectious acute pancreatitis, chronic pancreatitis, alcohol induced chronic pancreatitis, or biliar duct obstruction pancreatitis.

Also disclosed are reagents and tools useful for measuring Quiescin Q6 and optionally the one or more other biomarkers concerned herein.

Hence, disclosed is a protein, polypeptide or peptide array or microarray comprising (a) Quiescin Q6 and/or a fragment thereof, preferably a known quantity or concentration of Quiescin Q6 and/or fragment thereof; and (b) optionally and preferably, one or more other biomarkers, preferably a known quantity or concentration of said one or more other biomarkers.

Also disclosed is a binding agent array or microarray comprising: (a) one or more binding agents capable of specifically binding to Quiescin Q6 and/or to fragments thereof, preferably a known quantity or concentration of said binding agents; and (b) optionally and preferably, one or more binding agents capable of specifically binding to one or more other biomarkers, preferably a known quantity or concentration of said binding agents.

Also disclosed are kits as taught here above configured as portable devices, such as, for example, bed-side devices, for use at home or in clinical settings.

A related aspect thus provides a portable testing device capable of measuring the quantity of Quiescin Q6 and/or fragments thereof in a sample from a subject comprising: (i) optionally means for obtaining a sample from the subject, (ii) means for measuring the quantity of Quiescin Q6 and/or fragments in said sample, and (iii) means for visualising the quantity of Quiescin Q6 and/or fragments measured in the sample.

In an embodiment, the means of parts (ii) and (iii) may be the same, thus providing a portable testing device capable of measuring the quantity of Quiescin Q6 and/or fragments thereof in a sample from a subject comprising (i) means for obtaining a sample from the subject; and (ii) means for measuring the quantity of Quiescin Q6 and/or thereof in said sample and visualising the quantity of Quiescin Q6 and/or fragments measured in the sample.

In an embodiment of the testing device, said visualising means is capable of indicating whether the quantity of Quiescin Q6 and/or fragments thereof in the sample is above or below a certain threshold level and/or whether the quantity of Quiescin Q6 and/or fragments thereof in the sample deviates or not from a reference value of the quantity of Quiescin Q6 and/or fragments thereof, said reference value representing a known diagnosis, prediction and/or prognosis of impaired or aberrant beta cell activity due to a condition selected from the group consisting of: pre-onset type 1 diabetes mellitus (T1 D), pre-clinical T1D, early onset T1 D, emerging T1 D; pancreatitis such as acute pancreatitis, alcohol induced acute pancreatitis, infectious acute pancreatitis, chronic pancreatitis, alcohol induced chronic pancreatitis, or biliar duct obstruction pancreatitis. Hence, the portable testing device may suitably also comprise said reference value or means for establishing the reference value.

In an embodiment, the threshold level is chosen such that the quantity of Quiescin Q6 and/or fragments thereof in the sample above or below (depending on the marker and the disease or condition) said threshold level indicates that the subject has or is at risk of having the respective disease or condition or indicates a poor prognosis for such in the subject, and the quantity of Quiescin Q6 and/or fragments thereof in the sample below or above (depending on the disease or condition) said threshold level indicates that the subject does not have or is not at risk of having the diseases or conditions as taught herein or indicates a good prognosis for such in the subject.

In a preferred embodiment, said portable device can be used to monitor the pre-onset, onset, emergence, or development and/or progression of impaired or aberrant beta cell activity due to a condition selected from the group consisting of: pre-onset type 1 diabetes mellitus (T1D), pre-clinical T1D, early onset T1D, emerging T1D; pancreatitis such as: acute pancreatitis, alcohol induced acute pancreatitis, infectious acute pancreatitis, chronic pancreatitis, alcohol induced chronic pancreatitis, or biliar duct obstruction pancreatitis, by measuring the Quiescin Q6 level in a sample of the subject at regular time-intervals. Preferably, said measurements can be done daily, weekly, biweekly, monthly, 2, 3, 4, 5, or 6-monthly, yearly and the like, depending on the potential risk of the subject to develop clinical signs of any one of said conditions. The frequency of the measurements can be decided based on e.g. familial (genetic) predisposition or on patient (clinical) history and/or diet.

In an alternative embodiment of any one of the kits of the invention as defined herein, said impaired or aberrant beta cell activity due to a condition selected from the group consisting of: insulin resistance, exhaustion of beta cells, emerging or developing T2D.

In a further embodiment of the kits of the invention as defined herein, said impaired or aberrant beta cell activity due to pancreatic cancer or tumour formation.

Other aspects relate to the realisation that markers disclosed herein may be valuable targets for therapeutic and/or prophylactic interventions in diseases and conditions as taught herein.

Hence, also disclosed herein are any one and all of the following:
(1) an agent that is able to modulate the level and/or the activity of Quiescin Q6 nucleic acid or protein, or is able to interfere with its function for use as a medicament, preferably for use in the treatment of any one disease or condition as taught herein;
(2) use of an agent that is able to modulate the level and/or the activity of said one or more nucleic acids or proteins as defined in (1) above for the manufacture of a medicament for the treatment of conditions linked to impaired or aberrant beta cell activity selected from the group consisting of: pre-onset type 1 diabetes mellitus (T1D), pre-clinical T1D, early onset T1D, emerging T1D; pancreatitis such as acute pancreatitis, alcohol induced acute pancreatitis, infectious acute pancreatitis, chronic pancreatitis, alcohol induced chronic pancreatitis, or biliar duct obstruction pancreatitis; or use of an agent that is able to modulate the level and/or the activity of said one or more nucleic acids or proteins as defined in (1) above for the treatment of conditions linked to impaired or aberrant beta cell activity selected from the group consisting of: pre-onset type 1 diabetes mellitus (T1D), pre-clinical T1D, early onset T1D, emerging T1D; pancreatitis such as: acute pancreatitis, alcohol induced acute pancreatitis, infectious acute pancreatitis, chronic pancreatitis, alcohol induced chronic pancreatitis, or biliar duct obstruction pancreatitis;
(3) a method for treating conditions linked to impaired or aberrant beta cell activity selected from the group consisting of: pre-onset type 1 diabetes mellitus (T1D), pre-clinical T1D, early onset T1D, emerging T1D; pancreatitis such as: acute pancreatitis, alcohol induced acute pancreatitis, infectious acute pancreatitis, chronic pancreatitis, alcohol induced chronic pancreatitis, or biliar duct obstruction pancreatitis in a subject in need of such treatment, comprising administering to said subject a therapeutically or prophylactically effective amount of an agent that is able to modulate the level and/or the activity of said one or more nucleic acids or proteins as defined in (1) above, or is able to interfere with its function;
(4) The subject matter as set forth in any one of (1) to (3) above, wherein the agent is able to reduce or increase the level and/or the activity of said one or more nucleic acids or proteins as defined in (1) above.
(5) The subject matter as set forth in any one of (1) to (4) above, wherein said agent is able to specifically bind to said one or more nucleic acids or proteins as defined in (1) above and/or is able to interfere with the protein as defined in (1) above, or its activity.
(6) The subject matter as set forth in any one of (1) to (5) above, wherein said agent is an antibody or a fragment or derivative thereof; a polypeptide; a peptide; a peptidomimetic; a nanobody, an aptamer; a photoaptamer; or a chemical substance, preferably an organic molecule, more preferably a small organic molecule.
(7) The subject matter as set forth in any one of (1) to (4) above, wherein the agent is able to reduce or inhibit the expression of said one or more nucleic acids or proteins as defined in (1) above, preferably wherein said agent is an antisense agent; a ribozyme; or an agent capable of causing RNA interference.
(8) The subject matter as set forth in any one of (1) to (4) above, wherein said agent is able to reduce or inhibit the level and/or activity of said one or more nucleic acids or proteins as defined in (1) above, preferably wherein said agent is a recombinant or isolated deletion construct of the said one or more proteins as defined in (1) above polypeptide having a dominant negative activity over the native one or more proteins as defined in (1) above.
(9) An assay to select, from a group of test agents, a candidate agent potentially useful in the treatment of conditions linked to impaired or aberrant beta cell activity selected from the group consisting of: pre-onset type 1 diabetes mellitus (T1D), pre-clinical T1D, early onset T1D, emerging T1D; pancreatitis such as acute pancreatitis, alcohol induced acute pancreatitis, infectious acute pancreatitis, chronic pancreatitis, alcohol induced chronic pancreatitis, or biliar duct obstruction pancreatitis, said assay comprising determining whether a tested agent can modulate, such as increase or reduce the level and/or activity of said one or more nucleic acids or proteins as defined in (1) above.
(10) The assay as set forth in (9) above, further comprising use of the selected candidate agent for the preparation of a composition for administration to and monitoring the prophylactic and/or therapeutic effect thereof in a non-human animal model, preferably a non-human mammal model, of any one disease or condition as taught herein.
(11) The agent isolated by the assay as set forth in (10) above.
(12) A pharmaceutical composition or formulation comprising a prophylactically and/or therapeutically effective amount of one or more agents as set forth in any one of (1) to (8) or (10) above, or a pharmaceutically acceptable N-oxide form, addition salt, prodrug or solvate thereof, and further comprising one or more of pharmaceutically acceptable carriers.
(13) A method for producing the pharmaceutical composition or formulation as set forth in (12) above, comprising admixing said one or more agents with said one or more pharmaceutically acceptable carriers.

Said condition or disease as set forth in any one of (1) to (13) above may be particularly chosen from a condition linked to impaired or aberrant beta cell activity selected from the group consisting of: pre-onset type 1 diabetes mellitus (T1D), pre-clinical T1D, early onset T1D, emerging T1D; pancreatitis such as acute pancreatitis, alcohol induced acute pancreatitis, infectious acute pancreatitis, chronic pancreatitis, alcohol induced chronic pancreatitis, or biliar duct obstruction pancreatitis.

Alternatively, said condition or disease as set forth in any one of (1) to (13) above may be particularly chosen from a condition linked to impaired or aberrant beta cell activity selected from the group consisting of: insulin resistance, exhaustion of beta cells, emerging or developing T2D.

In a further embodiment of the invention, said condition or disease as set forth in any one of (1) to (13) above may be particularly chosen from a condition linked to impaired or aberrant beta cell activity selected from the group consisting of: pancreatic cancer or tumour formation.

Also contemplated is thus a method (a screening assay) for selecting an agent capable of specifically binding to Quiescin Q6 nucleic acids or proteins (e.g., nucleic acid such as gene, or protein) comprising: (a) providing one or more, preferably a plurality of, test binding agents; (b) selecting from the test binding agents of (a) those which bind to said one or more nucleic acids or proteins; and (c) counter-selecting (i.e., removing) from the test binding agents selected in (b) those which bind to any one or more other, unintended or undesired, targets.

Binding between test binding agents and said one or more nucleic acids or proteins may be advantageously tested by contacting (i.e., combining, exposing or incubating) said one or more nucleic acids or proteins with the test binding agents under conditions generally conducive for such binding. For example and without limitation, binding between test binding agents and said one or more nucleic acids or proteins may be suitably tested in vitro; or may be tested in host cells or host organisms comprising said one or more nucleic acids or proteins and exposed to or configured to express the test binding agents.

Without limitation, the binding or modulating agents may be capable of binding said one or more nucleic acids or proteins or modulating the activity and/or level of said one or more nucleic acids or proteins in vitro, in a cell, in an organ and/or in an organism.

In the screening assays as set forth in any one of (9) and (10) above, modulation of the activity and/or level of said one or more nucleic acids or proteins by test modulating agents may be advantageously tested by contacting (i.e., combining, exposing or incubating) said one or more nucleic acids or proteins (e.g., gene or protein) with the test modulating agents under conditions generally conducive for such modulation. By means of example and not limitation, where modulation of the activity and/or level of said one or more nucleic acids or proteins results from binding of the test modulating agents to said one or more nucleic acids or proteins, said conditions may be generally conducive for such binding. For example and without limitation, modulation of the activity and/or level of said one or more nucleic acids or proteins by test modulating agents may be suitably tested in vitro; or may be tested in host cells or host organisms comprising said one or more nucleic acids or proteins and exposed to or configured to express the test modulating agents.

As well contemplated are:
- Quiescin Q6 (e.g., nucleic acid such as a gene, or a polypeptide or protein) for use as a medicament, preferably for use in the treatment of conditions linked to impaired or aberrant beta cell activity selected from the group consisting of: pre-onset type 1 diabetes mellitus (T1 D), pre-clinical T1D, early onset T1D, emerging T1D; pancreatitis such as acute pancreatitis, alcohol induced acute pancreatitis, infectious acute pancreatitis, chronic pancreatitis, alcohol induced chronic pancreatitis, or biliar duct obstruction pancreatitis;
- use of said one or more Quiescin Q6 nucleic acids or proteins for the manufacture of a medicament for the treatment of conditions linked to impaired or aberrant beta cell activity selected from the group consisting of: pre-onset type 1 diabetes mellitus (T1 D), pre-clinical T1D, early onset T1D, emerging T1D; pancreatitis such as acute pancreatitis, alcohol induced acute pancreatitis, infectious acute pancreatitis, chronic pancreatitis, alcohol induced chronic pancreatitis, or biliar duct obstruction pancreatitis;
- use of said one or more Quiescin Q6 nucleic acids or proteins for the treatment of conditions linked to impaired or aberrant beta cell activity selected from the group consisting of: pre-onset type 1 diabetes mellitus (T1 D), pre-clinical T1 D, early onset T1D, emerging T1D; pancreatitis such as acute pancreatitis, alcohol induced acute pancreatitis, infectious acute pancreatitis, chronic pancreatitis, alcohol induced chronic pancreatitis, or biliar duct obstruction pancreatitis;
- a method for treating a condition linked to impaired or aberrant beta cell activity selected from the group consisting of: pre-onset type 1 diabetes mellitus (T1D), pre-clinical T1D, early onset T1D, emerging T1D; pancreatitis such as acute pancreatitis, alcohol induced acute pancreatitis, infectious acute pancreatitis, chronic pancreatitis, alcohol induced chronic pancreatitis, or biliar duct obstruction pancreatitis in a subject in need of such treatment, comprising administering to said subject a therapeutically or prophylactically effective amount of said one or more Quiescin Q6 nucleic acids or proteins.

Alternatively, in any one of the embodiments above, said conditions linked to impaired or aberrant beta cell activity can be selected from the group consisting of: insulin resistance, exhaustion of beta cells, emerging or developing T2D.

In a further embodiment of the invention, said conditions linked to impaired or aberrant beta cell activity can be selected from the group consisting of: pancreatic cancer or tumour formation.

The goal of the treatment or therapy with Quiescin Q6 protein of nucleic acid or agents modulating its expression or activity is to restore impaired or normalise aberrant beta cell activity in the subject.

Reference throughout this specification to "diseases and/or conditions" encompasses any such diseases and conditions as disclosed herein insofar consistent with the context of a particular recitation, more specifically but without limitation including conditions linked to impaired or aberrant beta cell activity selected from the group consisting of: pre-onset type 1 diabetes mellitus (T1D), pre-clinical T1D, early onset T1D, emerging T1D; pancreatitis such as acute pancreatitis, alcohol induced acute pancreatitis, infectious acute pancreatitis, chronic pancreatitis, alcohol induced chronic pancreatitis, or biliar duct obstruction pancreatitis.

Alternatively, said reference "diseases and/or conditions" encompasses any such diseases and conditions as disclosed herein insofar consistent with the context of a particular recitation, more specifically but without limitation including conditions linked to impaired or aberrant beta cell activity selected from the group consisting of: insulin resistance, exhaustion of beta cells, emerging or developing T2D.

In a further embodiment of the invention, said reference "diseases and/or conditions" encompasses any such diseases and conditions as disclosed herein insofar consistent with the context of a particular recitation, more specifically but without limitation including conditions linked to impaired or aberrant beta cell activity due to pancreatic cancer or tumour formation.

The above and further aspects and preferred embodiments of the invention are described in the following sections and in the appended claims. The subject matter of the appended claims is hereby specifically incorporated in this specification.

### BRIEF DESCRIPTION OF FIGURES

**Figure 1****:** illustrates sequences of isoform 1 (**A**) (SEQ ID NO: 1) and isoform 2 (**B**) (SEQ ID NO: 2) of Quiescin Q6.
**Figure 2****:** illustrates the differences between isoforms 1 (SEQ ID NO: 1) and 2 (SEQ ID NO:2) of Quiescin Q6. The C-terminal portion missing in isoform 2 is indicated in small letters. Also indicated is the selected MASSterclass quantified peptide (pept110 - bold, double underlined, SEQ ID NO: 3). The MASSterclass peptide can quantify both isoforms of Quiescin Q6.
**Figure 3****:** Box and whisker plots showing plasma Quiescin Q6 levels as measured by MASSterclass over different populations: CHF = chronic stable heart failure; PE = pregnant women at 22 weeks of gestation who will develop pre-eclampsia later during the pregnancy; SIRS = systemic inflammatory response syndrome.

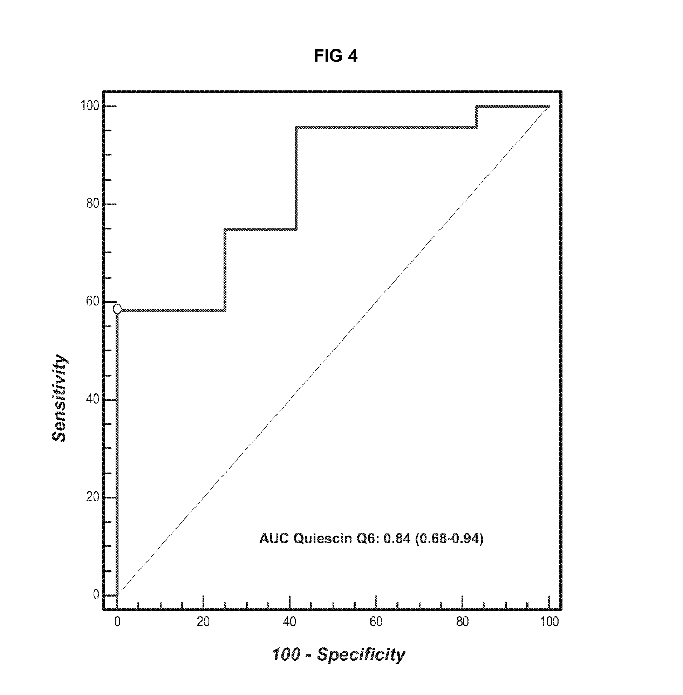

**Figure 4****:** Receiver operating characteristic curve of Quiescin Q6 for discriminating pancreas cancer from chronic pancreatitis. Calculated median area under the curve (AUC) and 95% confidence intervals are shown.

### DETAILED DESCRIPTION OF THE INVENTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The term also encompasses "consisting of" and "consisting essentially of".

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of and from the specified value, in particular variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed. Whereas the term "one or more", such as one or more members of a group of members, is clear per se, by means of further exemplification, the term encompasses inter alia a reference to any one of said members, or to any two or more of said members, such as, e.g., ≥ 3, ≥ 4, ≥ 5, ≥ 6 or ≥ 7 etc. of said members, and up to all said members.

All documents cited in the present specification are hereby incorporated by reference in their entirety.

Unless otherwise specified, all terms used in disclosing the invention, including technical and scientific terms, have the meaning as commonly understood by one of ordinary skill in the art to which this invention belongs. By means of further guidance, term definitions may be included to better appreciate the teaching of the present invention.

The inventors identified Quiescin Q6 as a valuable biomarker particularly for measuring abnormal beta cell mass and/or activity in subjects.

Abnormal beta cell mass and/or activity can be linked to various conditions such as those selected from the group consisting of: pre-onset type 1 diabetes mellitus (T1D), pre-clinical T1D, early onset T1D, emerging T1D; pancreatitis such as acute pancreatitis, alcohol induced acute pancreatitis, infectious acute pancreatitis, chronic pancreatitis, alcohol induced chronic pancreatitis, or biliar duct obstruction pancreatitis.

Alternatively, said conditions can be selected from the group consisting of: insulin resistance, exhaustion of beta cells, emerging or developing T2D.

In a further embodiment of the invention, said conditions can be pancreatic cancer or tumour formation.

The term "biomarker" is widespread in the art and may broadly denote a biological molecule and/or a detectable portion thereof whose qualitative and/or quantitative evaluation in a subject is predictive or informative (e.g., predictive, diagnostic and/or prognostic) with respect to one or more aspects of the subject's phenotype and/or genotype, such as, for example, with respect to the status of the subject as to a given disease or condition. Preferably, biomarkers as intended herein are peptide-, polypeptide- and/or protein-based. The terms "biomarker" and "marker" may be used interchangeably herein.

The terms "predicting" or "prediction", "diagnosing" or "diagnosis" and "prognosticating" or "prognosis" are commonplace and well-understood in medical and clinical practice. It shall be understood that the phrase "a method for the diagnosis, prediction and/or prognosis" a given disease or condition may also be interchanged with phrases such as "a method for diagnosing, predicting and/or prognosticating" of said disease or condition or "a method for making (or determining or establishing) the diagnosis, prediction and/or prognosis" of said disease or condition, or the like.

By means of further explanation and without limitation, "predicting" or "prediction" generally refer to an advance declaration, indication or foretelling of a disease or condition in a subject not (yet) having said disease or condition. For example, a prediction of a disease or condition in a subject may indicate a probability, chance or risk that the subject will develop said disease or condition, for example within a certain time period or by a certain age. Said probability, chance or risk may be indicated inter alia as an absolute value, range or statistics, or may be indicated relative to a suitable control subject or subject population (such as, e.g., relative to a general, normal or healthy subject or subject population). Hence, the probability, chance or risk that a subject will develop a disease or condition may be advantageously indicated as increased or decreased, or as fold-increased or fold-decreased relative to a suitable control subject or subject population. As used herein, the term "prediction" of the conditions or diseases as taught herein in a subject may also particularly mean that the subject has a 'positive' prediction of such, i.e., that the subject is at risk of having such (e.g., the risk is significantly increased vis-à-vis a control subject or subject population). The term "prediction of no" diseases or conditions as taught herein as described herein in a subject may particularly mean that the subject has a 'negative' prediction of such, i.e., that the subject's risk of having such is not significantly increased vis-à-vis a control subject or subject population.

The terms "diagnosing" or "diagnosis" generally refer to the process or act of recognising, deciding on or concluding on a disease or condition in a subject on the basis of symptoms and signs and/or from results of various diagnostic procedures (such as, for example, from knowing the presence, absence and/or quantity of one or more biomarkers characteristic of the diagnosed disease or condition). As used herein, "diagnosis of" the diseases or conditions as taught herein in a subject may particularly mean that the subject has such, hence, is diagnosed as having such. "Diagnosis of no" diseases or conditions as taught herein in a subject may particularly mean that the subject does not have such, hence, is diagnosed as not having such. A subject may be diagnosed as not having such despite displaying one or more conventional symptoms or signs reminiscent of such.

The terms "prognosticating" or "prognosis" generally refer to an anticipation on the progression of a disease or condition and the prospect (e.g., the probability, duration, and/or extent) of recovery. A good prognosis of the diseases or conditions taught herein may generally encompass anticipation of a satisfactory partial or complete recovery from the diseases or conditions, preferably within an acceptable time period. A good prognosis of such may more commonly encompass anticipation of not further worsening or aggravating of such, preferably within a given time period. A poor prognosis of the diseases or conditions as taught herein may generally encompass anticipation of a substandard recovery and/or unsatisfactorily slow recovery, or to substantially no recovery or even further worsening of such.

The term "subject" or "patient" as used herein typically denotes humans, but may also encompass reference to non-human animals, preferably warm-blooded animals, more preferably viviparous animals, even more preferably mammals, such as, e.g., non-human primates, rodents, canines, felines, equines, ovines, porcines, and the like. Particularly intended are female subjects, more particularly pregnant or postpartum female subjects.

The terms "sample" or "biological sample" as used herein include any biological specimen obtained from a subject. Samples may include, without limitation, whole blood, plasma, serum, red blood cells, white blood cells (e.g., peripheral blood mononuclear cells), saliva, urine, stool (i.e., faeces), tears, sweat, sebum, nipple aspirate, ductal lavage, tumour exudates, synovial fluid, cerebrospinal fluid, lymph, fine needle aspirate, amniotic fluid, any other bodily fluid, cell lysates, cellular secretion products, inflammation fluid, semen and vaginal secretions. Preferred samples may include ones comprising any one or more markers as taught herein protein in detectable quantities. In preferred embodiments, the sample may be whole blood or a fractional component thereof such as, e.g., plasma, serum, or a cell pellet. Preferably the sample is readily obtainable by minimally invasive methods, allowing to remove or isolate said sample from the subject. Samples may also include tissue samples and biopsies, tissue homogenates and the like, such as pancreatic tissue or biopsies, pancreatic islet biopsies or beta cell biopsies. Preferably, the sample used to detect the levels of any one or more markers as taught herein is blood serum or blood plasma. Also preferably, said sample is urine. The term "plasma" generally denotes the substantially colourless watery fluid of the blood that contains no cells, but in which the blood cells (erythrocytes, leukocytes, thrombocytes, etc.) are normally suspended, containing nutrients, sugars, proteins, minerals, enzymes, etc.. The term serum comprises the component of blood which is collected after coagulation..

A molecule or analyte such as a protein, polypeptide or peptide, or a group of two or more molecules or analytes such as two or more proteins, polypeptides or peptides, is "measured" in a sample when the presence or absence and/or quantity of said molecule or analyte or of said group of molecules or analytes is detected or determined in the sample, preferably substantially to the exclusion of other molecules and analytes.

The terms "quantity", "amount" and "level" are synonymous and generally well-understood in the art. The terms as used herein may particularly refer to an absolute quantification of a molecule or an analyte in a sample, or to a relative quantification of a molecule or analyte in a sample, i.e., relative to another value such as relative to a reference value as taught herein, or to a range of values indicating a base-line expression of the biomarker. These values or ranges can be obtained from a single patient or from a group of patients.

An absolute quantity of a molecule or analyte in a sample may be advantageously expressed as weight or as molar amount, or more commonly as a concentration, e.g., weight per volume or mol per volume.

A relative quantity of a molecule or analyte in a sample may be advantageously expressed as an increase or decrease or as a fold-increase or fold-decrease relative to said another value, such as relative to a reference value as taught herein. Performing a relative comparison between first and second parameters (e.g., first and second quantities) may but need not require to first determine the absolute values of said first and second parameters. For example, a measurement method can produce quantifiable readouts (such as, e.g., signal intensities) for said first and second parameters, wherein said readouts are a function of the value of said parameters, and wherein said readouts can be directly compared to produce a relative value for the first parameter vs. the second parameter, without the actual need to first convert the readouts to absolute values of the respective parameters.

As used herein, the reference to any one marker (biomarker), nucleic acid, peptide, polypeptide or protein corresponds to the marker, nucleic acid, peptide, polypeptide or protein commonly known under the respective designations in the art. The terms encompass such markers, nucleic acids, proteins and polypeptides of any organism where found, and particularly of animals, preferably warm-blooded animals, more preferably vertebrates, yet more preferably mammals, including humans and non-human mammals, still more preferably of humans. The terms particularly encompass such markers, nucleic acids, proteins and polypeptides with a native sequence, i.e., ones of which the primary sequence is the same as that of the markers, nucleic acids, proteins and polypeptides found in or derived from nature. A skilled person understands that native sequences may differ between different species due to genetic divergence between such species. Moreover, native sequences may differ between or within different individuals of the same species due to normal genetic diversity (variation) within a given species. Also, native sequences may differ between or even within different individuals of the same species due to post-transcriptional or post-translational modifications. Any such variants or isoforms of markers, nucleic acids, proteins and polypeptides are intended herein. Accordingly, all sequences of markers, nucleic acids, proteins and polypeptides found in or derived from nature are considered "native". The terms encompass the markers, nucleic acids, proteins and polypeptides when forming a part of a living organism, organ, tissue or cell, when forming a part of a biological sample, as well as when at least partly isolated from such sources. The terms also encompass proteins and polypeptides when produced by recombinant or synthetic means.

The reference herein to any biomolecule, such as a marker (biomarker), peptide, polypeptide or protein may also encompass fragments thereof. Hence, the reference herein to measuring (or measuring the quantity of) any one marker or biomolecule may encompass measuring the marker or biomolecule, such as, e.g., measuring the mature and/or the processed soluble/secreted form (e.g. plasma circulating form) of the marker or biomolecule and/or measuring one or more fragments thereof.

For example, any marker or biomolecule and/or one or more fragments thereof may be measured collectively, such that the measured quantity corresponds to the sum amounts of the collectively measured species. In another example, any marker or biomolecule and/or one or more fragments thereof may be measured each individually. Preferably, said fragment may be a plasma circulating (i.e., not cell- or membrane-bound) form. Without being bound by any theory, such circulating forms can be derived from full-length markers or biomolecules through natural processing, or can be resulting from known degradation processes occurring in a sample. In certain situations, the circulating form can also be the full-length marker or biomolecule, which is found to be circulating in the plasma. Said "circulating form" can thus be any marker or biomolecule or any processed soluble form thereof or fragments of either one, that is circulating in the sample, i.e. which is not bound to a cell- or membrane fraction of said sample.

Unless otherwise apparent from the context, reference herein to any biomolecule such as a marker, peptide, polypeptide or protein encompasses such from any organism where found, and particularly preferably from animals, preferably warm-blooded animals, more preferably vertebrates, even preferably mammals, including humans and non-human mammals, still more preferably from humans.

Further, unless otherwise apparent from the context, reference herein to any marker, peptide, polypeptide or protein and fragments thereof may generally also encompass modified forms of said marker, peptide, polypeptide or protein and fragments such as bearing post-expression modifications including, for example, phosphorylation, glycosylation, lipidation, methylation, cysteinylation, sulphonation, glutathionylation, acetylation, oxidation of methionine to methionine sulphoxide or methionine sulphone, and the like.

In an embodiment, any marker, peptide, polypeptide or protein and fragments thereof, or other biomarkers as employed herein and fragments thereof, may be human, i.e., their primary sequence may be the same as a corresponding primary sequence of or present in a naturally occurring human markers, peptides, polypeptides or proteins. Hence, the qualifier "human" in this connection relates to the primary sequence of the respective markers, peptides, polypeptides, proteins or fragments, rather than to their origin or source. For example, such markers, peptides, polypeptides, proteins or fragments may be present in or isolated from samples of human subjects or may be obtained by other means (e.g., by recombinant expression, cell-free translation or non-biological peptide synthesis).

The term "fragment" of a protein, polypeptide or peptide generally refers to N-terminally and/or C-terminally deleted or truncated forms of said protein, polypeptide or peptide. The term encompasses fragments arising by any mechanism, such as, without limitation, by alternative translation, exo- and/or endo-proteolysis and/or degradation of said peptide, polypeptide or protein, such as, for example, in vivo or in vitro, such as, for example, by physical, chemical and/or enzymatic proteolysis. Without limitation, a fragment of a protein, polypeptide or peptide may represent at least about 5%, or at least about 10%, e.g., ≥ 20%, ≥ 30% or ≥ 40%, such as ≥ 50%, e.g., ≥ 60%, ≥ 70% or ≥ 80%, or even ≥ 90% or ≥ 95% of the amino acid sequence of said protein, polypeptide or peptide.

For example, a fragment may include a sequence of ≥ 5 consecutive amino acids, or ≥ 10 consecutive amino acids, or ≥ 20 consecutive amino acids, or ≥ 30 consecutive amino acids, e.g., ≥ 40 consecutive amino acids, such as for example ≥ 50 consecutive amino acids, e.g., ≥ 60, ≥ 70, ≥ 80, ≥ 90, ≥ 100, ≥ 200, ≥ 300, ≥ 400, ≥ 500 or ≥ 600 consecutive amino acids of the corresponding full length protein.

In an embodiment, a fragment may be N-terminally and/or C-terminally truncated by between 1 and about 20 amino acids, such as, e.g., by between 1 and about 15 amino acids, or by between 1 and about 10 amino acids, or by between 1 and about 5 amino acids, compared to the corresponding mature, full-length protein or its soluble or plasma circulating form.

In an embodiment, fragments of a given protein, polypeptide or peptide may be achieved by in vitro proteolysis of said protein, polypeptide or peptide to obtain advantageously detectable peptide(s) from a sample. For example, such proteolysis may be effected by suitable physical, chemical and/or enzymatic agents, e.g., proteinases, preferably endoproteinases, i.e., protease cleaving internally within a protein, polypeptide or peptide chain. A non-limiting list of suitable endoproteinases includes serine proteinases (EC 3.4.21), threonine proteinases (EC 3.4.25), cysteine proteinases (EC 3.4.22), aspartic acid proteinases (EC 3.4.23), metalloproteinases (EC 3.4.24) and glutamic acid proteinases. Exemplary non-limiting endoproteinases include trypsin, chymotrypsin, elastase, Lysobacter enzymogenes endoproteinase Lys-C, Staphylococcus aureus endoproteinase Glu-C (endopeptidase V8) or Clostridium histolyticum endoproteinase Arg-C (clostripain). Further known or yet to be identified enzymes may be used; a skilled person can choose suitable protease(s) on the basis of their cleavage specificity and frequency to achieve desired peptide forms. Preferably, the proteolysis may be effected by endopeptidases of the trypsin type (EC 3.4.21.4), preferably trypsin, such as, without limitation, preparations of trypsin from bovine pancreas, human pancreas, porcine pancreas, recombinant trypsin, Lys-acetylated trypsin, trypsin in solution, trypsin immobilised to a solid support, etc. Trypsin is particularly useful, inter alia due to high specificity and efficiency of cleavage. The invention also contemplates the use of any trypsin-like protease, i.e., with a similar specificity to that of trypsin. Otherwise, chemical reagents may be used for proteolysis. For example, CNBr can cleave at Met; BNPS-skatole can cleave at Trp. The conditions for treatment, e.g., protein concentration, enzyme or chemical reagent concentration, pH, buffer, temperature, time, can be determined by the skilled person depending on the enzyme or chemical reagent employed.

Also provided is thus an isolated fragment of Quiescin Q6 as defined here above. Such fragments may give useful information about the presence and quantity of said marker, peptides, polypeptides or proteins in biological samples, whereby the detection of said fragments is of interest. Hence, the herein disclosed fragments of said markers, peptides, polypeptides or proteins are useful biomarkers.

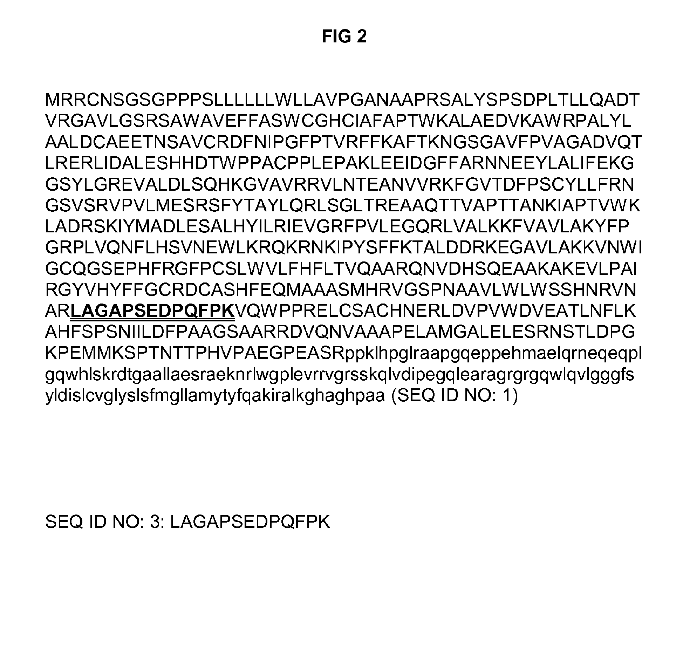

Preferred fragments may comprise, consist essentially of or consist of the sequence as set forth in SEQ ID NO: 3 depicted in Figure 2, which was used in the examples to provide information on the respective markers.

The term "isolated" with reference to a particular component (such as for instance, a protein, polypeptide, peptide or fragment thereof) generally denotes that such component exists in separation from - for example, has been separated from or prepared in separation from - one or more other components of its natural environment. For instance, an isolated human or animal protein, polypeptide, peptide or fragment exists in separation from a human or animal body where it occurs naturally.

The term "isolated" as used herein may preferably also encompass the qualifier "purified". As used herein, the term "purified" with reference to protein(s), polypeptide(s), peptide(s) and/or fragment(s) thereof does not require absolute purity. Instead, it denotes that such protein(s), polypeptide(s), peptide(s) and/or fragment(s) is (are) in a discrete environment in which their abundance (conveniently expressed in terms of mass or weight or concentration) relative to other proteins is greater than in a biological sample. A discrete environment denotes a single medium, such as for example a single solution, gel, precipitate, lyophilisate, etc. Purified peptides, polypeptides or fragments may be obtained by known methods including, for example, laboratory or recombinant synthesis, chromatography, preparative electrophoresis, centrifugation, precipitation, affinity purification, etc.

Purified protein(s), polypeptide(s), peptide(s) and/or fragment(s) may preferably constitute by weight ≥ 10%, more preferably ≥ 50%, such as ≥ 60%, yet more preferably ≥ 70%, such as ≥ 80%, and still more preferably ≥ 90%, such as ≥ 95%, ≥ 96%, ≥ 97%, ≥ 98%, ≥ 99% or even 100%, of the protein content of the discrete environment. Protein content may be determined, e.g., by the Lowry method (Lowry et al. 1951. J Biol Chem 193: 265), optionally as described by Hartree 1972 (Anal Biochem 48: 422-427). Also, purity of peptides or polypeptides may be determined by SDS-PAGE under reducing or non-reducing conditions using Coomassie blue or, preferably, silver stain.

Further disclosed are any isolated marker, peptide, polypeptide or protein and fragments thereof as taught herein comprising a detectable label. This facilitates ready detection of such fragments. The term "label" as used throughout this specification refers to any atom, molecule, moiety or biomolecule that can be used to provide a detectable and preferably quantifiable read-out or property, and that can be attached to or made part of an entity of interest, such as a peptide or polypeptide or a specific-binding agent. Labels may be suitably detectable by mass spectrometric, spectroscopic, optical, colourimetric, magnetic, photochemical, biochemical, immunochemical or chemical means. Labels include without limitation dyes; radiolabels such as 32P, 33P, 35S, 125I, 131I; electron-dense reagents; enzymes (e.g. , horse-radish phosphatise or alkaline phosphatise as commonly used in immunoassays); binding moieties such as biotin-streptavidin; haptens such as digoxigenin; luminogenic, phosphorescent or fluorogenic moieties; mass tags; and fluorescent dyes alone or in combination with moieties that can suppress or shift emission spectra by fluorescence resonance energy transfer (FRET).

For example, the label may be a mass-altering label. Preferably, a mass-altering label may involve the presence of a distinct stable isotope in one or more amino acids of the peptide vis-à-vis its corresponding non-labelled peptide. Mass-labelled peptides are particularly useful as positive controls, standards and calibrators in mass spectrometry applications. In particular, peptides including one or more distinct isotopes are chemically alike, separate chromatographically and electrophoretically in the same manner and also ionise and fragment in the same way. However, in a suitable mass analyser such peptides and optionally select fragmentation ions thereof will display distinguishable m/z ratios and can thus be discriminated. Examples of pairs of distinguishable stable isotopes include H and D, 12C and 13C, 14N and 15N or 160 and 180. Usually, peptides and proteins of biological samples analysed in the present invention may substantially only contain common isotopes having high prevalence in nature, such as for example H, 12C, 14N and 160. In such case, the mass-labelled peptide may be labelled with one or more uncommon isotopes having low prevalence in nature, such as for instance D, 13C, 15N and/or 180. It is also conceivable that in cases where the peptides or proteins of a biological sample would include one or more uncommon isotopes, the mass-labelled peptide may comprise the respective common isotope(s).

Isotopically-labelled synthetic peptides may be obtained inter alia by synthesising or recombinantly producing such peptides using one or more isotopically-labelled amino acid substrates, or by chemically or enzymatically modifying unlabelled peptides to introduce thereto one or more distinct isotopes. By means of example and not limitation, D-labelled peptides may be synthesised or recombinantly produced in the presence of commercially available deuterated L-methionine CH3-S-CD2CD2-CH(NH2)-COOH or deuterated arginine H2NC(=NH)-NH-(CD2)3-CD(NH2)-COOH. It shall be appreciated that any amino acid of which deuterated or 15N- or 13C-containing forms exist may be considered for synthesis or recombinant production of labelled peptides. In another non-limiting example, a peptide may be treated with trypsin in H216O or H218O, leading to incorporation of two oxygens (160 or 180, respectively) at the COOH-termini of said peptide (e.g., US 2006/105415).

Accordingly, also contemplated is the use of any (isolated) marker, peptide, polypeptide or protein and fragments thereof as taught herein, optionally comprising a detectable label, as (positive) controls, standards or calibators in qualitative or quantitative detection assays (measurement methods) of said marker, peptide, polypeptide or protein and fragments thereof, and particularly in such methods for the diagnosis, prediction, prognosis and/or monitoring the diseases or conditions as taught herein in subjects. The markers, proteins, polypeptides or peptides may be supplied in any form, inter alia as precipitate, vacuum-dried, lyophilisate, in solution as liquid or frozen, or covalently or non-covalently immobilised on solid phase, such as for example, on solid chromatographic matrix or on glass or plastic or other suitable surfaces (e.g., as a part of peptide arrays and microarrays). The peptides may be readily prepared, for example, isolated from natural sources, or prepared recombinantly or synthetically.

Further disclosed are binding agents capable of specifically binding to any one or more (isolated) markers, peptides, polypeptides or proteins and fragments thereof as taught herein. Also disclosed are binding agents capable of specifically binding to only one of (isolated) markers, peptides, polypeptides or proteins and fragments thereof as taught herein. Binding agents as intended throughout this specification may include inter alia an antibody, aptamer, photoaptamer, protein, peptide, peptidomimetic or a small molecule.

The term "specifically bind" as used throughout this specification means that an agent (denoted herein also as "specific-binding agent") binds to one or more desired molecules or analytes, such as to one or more proteins, polypeptides or peptides of interest or fragments thereof substantially to the exclusion of other molecules which are random or unrelated, and optionally substantially to the exclusion of other molecules that are structurally related. The term "specifically bind" does not necessarily require that an agent binds exclusively to its intended target(s). For example, an agent may be said to specifically bind to protein(s) polypeptide(s), peptide(s) and/or fragment(s) thereof of interest if its affinity for such intended target(s) under the conditions of binding is at least about 2-fold greater, preferably at least about 5-fold greater, more preferably at least about 10-fold greater, yet more preferably at least about 25-fold greater, still more preferably at least about 50-fold greater, and even more preferably at least about 100-fold or more greater, than its affinity for a non-target molecule.

Preferably, the agent may bind to its intended target(s) with affinity constant (K_{A}) of such binding K_{A} ≥ 1×10⁶ M⁻¹, more preferably K_{A} ≥ 1×10⁷ M⁻¹, yet more preferably K_{A} ≥ 1×10⁸ M⁻¹, even more preferably K_{A} ≥ 1×10⁹ M⁻¹, and still more preferably K_{A}≥ 1×10¹⁰ M⁻¹ or K_{A} ≥ 1×10¹¹ M⁻¹, wherein K_{A} = [SBA_T]/[SBA][T], SBA denotes the specific-binding agent, T denotes the intended target. Determination of K_{A} can be carried out by methods known in the art, such as for example, using equilibrium dialysis and Scatchard plot analysis.

Specific binding agents as used throughout this specification may include inter alia an antibody, aptamer, photoaptamer, protein, peptide, peptidomimetic or a small molecule.

As used herein, the term "antibody" is used in its broadest sense and generally refers to any immunologic binding agent. The term specifically encompasses intact monoclonal antibodies, polyclonal antibodies, multivalent (e.g., 2-, 3- or more-valent) and/or multi-specific antibodies (e.g., bi- or more-specific antibodies) formed from at least two intact antibodies, and antibody fragments insofar they exhibit the desired biological activity (particularly, ability to specifically bind an antigen of interest), as well as multivalent and/or multi-specific composites of such fragments. The term "antibody" is not only inclusive of antibodies generated by methods comprising immunisation, but also includes any polypeptide, e.g., a recombinantly expressed polypeptide, which is made to encompass at least one complementarity-determining region (CDR) capable of specifically binding to an epitope on an antigen of interest. Hence, the term applies to such molecules regardless whether they are produced in vitro or in vivo.

An antibody may be any of IgA, IgD, IgE, IgG and IgM classes, and preferably IgG class antibody. An antibody may be a polyclonal antibody, e.g., an antiserum or immunoglobulins purified there from (e.g., affinity-purified). An antibody may be a monoclonal antibody or a mixture of monoclonal antibodies. Monoclonal antibodies can target a particular antigen or a particular epitope within an antigen with greater selectivity and reproducibility. By means of example and not limitation, monoclonal antibodies may be made by the hybridoma method first described by Kohler et al. 1975 (Nature 256: 495), or may be made by recombinant DNA methods (e.g., as in US 4,816,567). Monoclonal antibodies may also be isolated from phage antibody libraries using techniques as described by Clackson et al. 1991 (Nature 352: 624-628) and Marks et al. 1991 (J Mol Biol 222: 581-597), for example.

Antibody binding agents may be antibody fragments. "Antibody fragments" comprise a portion of an intact antibody, comprising the antigen-binding or variable region thereof. Examples of antibody fragments include Fab, Fab', F(ab')2, Fv and scFv fragments; diabodies; linear antibodies; single-chain antibody molecules; and multivalent and/or multispecific antibodies formed from antibody fragment(s), e.g., dibodies, tribodies, and multibodies. The above designations Fab, Fab', F(ab')2, Fv, scFv etc. are intended to have their art-established meaning.

The term antibody includes antibodies originating from or comprising one or more portions derived from any animal species, preferably vertebrate species, including, e.g., birds and mammals. Without limitation, the antibodies may be chicken, turkey, goose, duck, guinea fowl, quail or pheasant. Also without limitation, the antibodies may be human, murine (e.g., mouse, rat, etc.), donkey, rabbit, goat, sheep, guinea pig, camel (e.g., Camelus bactrianus and Camelus dromaderius), Ilama (e.g., Lama paccos, Lama glama or Lama vicugna) or horse.

A skilled person will understand that an antibody can include one or more amino acid deletions, additions and/or substitutions (e.g., conservative substitutions), insofar such alterations preserve its binding of the respective antigen. An antibody may also include one or more native or artificial modifications of its constituent amino acid residues (e.g., glycosylation, etc.).

Methods of producing polyclonal and monoclonal antibodies as well as fragments thereof are well known in the art, as are methods to produce recombinant antibodies or fragments thereof (see for example, Harlow and Lane, "Antibodies: A Laboratory Manual", Cold Spring Harbour Laboratory, New York, 1988; Harlow and Lane, "Using Antibodies: A Laboratory Manual", Cold Spring Harbour Laboratory, New York, 1999, ISBN 0879695447; "Monoclonal Antibodies: A Manual of Techniques", by Zola, ed., CRC Press 1987, ISBN 0849364760; "Monoclonal Antibodies: A Practical Approach", by Dean & Shepherd, eds., Oxford University Press 2000, ISBN 0199637229; Methods in Molecular Biology, vol. 248: "Antibody Engineering: Methods and Protocols", Lo, ed., Humana Press 2004, ISBN 1588290921).

The term "aptamer" refers to single-stranded or double-stranded oligo-DNA, oligo-RNA or oligo-DNA/RNA or any analogue thereof, that can specifically bind to a target molecule such as a peptide. Advantageously, aptamers can display fairly high specificity and affinity (e.g., KA in the order 1 109 M-1) for their targets. Aptamer production is described inter alia in US 5,270,163; Ellington & Szostak 1990 (Nature 346: 818-822); Tuerk & Gold 1990 (Science 249: 505-510); or "The Aptamer Handbook: Functional Oligonucleotides and Their Applications", by Klussmann, ed., Wiley-VCH 2006, ISBN 3527310592, incorporated by reference herein. The term "photoaptamer" refers to an aptamer that contains one or more photoreactive functional groups that can covalently bind to or crosslink with a target molecule. The term "peptidomimetic" refers to a non-peptide agent that is a topological analogue of a corresponding peptide. Methods of rationally designing peptidomimetics of peptides are known in the art. For example, the rational design of three peptidomimetics based on the sulphated 8-mer peptide CCK26-33, and of two peptidomimetics based on the 11-mer peptide Substance P, and related peptidomimetic design principles, are described in Horwell 1995 (Trends Biotechnol 13: 132-134).

The term "small molecule" refers to compounds, preferably organic compounds, with a size comparable to those organic molecules generally used in pharmaceuticals. The term excludes biological macromolecules (e.g., proteins, nucleic acids, etc.). Preferred small organic molecules range in size up to about 5000 Da, e.g., up to about 4000, preferably up to 3000 Da, more preferably up to 2000 Da, even more preferably up to about 1000 Da, e.g., up to about 900, 800, 700, 600 or up to about 500 Da.

Hence, also disclosed are methods for immunising animals, e.g., non-human animals such as laboratory or farm, animals using (i.e., using as the immunising antigen) any one or more (isolated) markers, peptides, polypeptides or proteins and fragments thereof as taught herein, optionally attached to a presenting carrier. Immunisation and preparation of antibody reagents from immune sera is well-known per se and described in documents referred to elsewhere in this specification. The animals to be immunised may include any animal species, preferably warm-blooded species, more preferably vertebrate species, including, e.g., birds and mammals. Without limitation, the antibodies may be chicken, turkey, goose, duck, guinea fowl, quail or pheasant. Also without limitation, the antibodies may be human, murine (e.g., mouse, rat, etc.), donkey, rabbit, goat, sheep, guinea pig, camel, Ilama or horse. The term "presenting carrier" or "carrier" generally denotes an immunogenic molecule which, when bound to a second molecule, augments immune responses to the latter, usually through the provision of additional T cell epitopes. The presenting carrier may be a (poly)peptidic structure or a non-peptidic structure, such as inter alia glycans, polyethylene glycols, peptide mimetics, synthetic polymers, etc. Exemplary non-limiting carriers include human Hepatitis B virus core protein, multiple C3d domains, tetanus toxin fragment C or yeast Ty particles.

Immune sera obtained or obtainable by immunisation as taught herein may be particularly useful for generating antibody reagents that specifically bind to any one or more (isolated) markers, peptides, polypeptides or proteins and fragments thereof disclosed herein.

Any existing, available or conventional separation, detection and quantification methods can be used herein to measure the presence or absence (e.g., readout being present vs. absent; or detectable amount vs. undetectable amount) and/or quantity (e.g., readout being an absolute or relative quantity, such as, for example, absolute or relative concentration) of markers, peptides, polypeptides, proteins and/or fragments thereof and optionally of the one or more other biomarkers or fragments thereof in samples (any molecules or analytes of interest to be so-measured in samples, including any one or more markers, peptides, polypeptides, proteins and fragments thereof as taught herein, may be herein below referred to collectively as biomarkers).

For example, such methods may include immunoassay methods, mass spectrometry analysis methods, or chromatography methods, or combinations thereof.

The term "immunoassay" generally refers to methods known as such for detecting one or more molecules or analytes of interest in a sample, wherein specificity of an immunoassay for the molecule(s) or analyte(s) of interest is conferred by specific binding between a specific-binding agent, commonly an antibody, and the molecule(s) or analyte(s) of interest. Immunoassay technologies include without limitation direct ELISA (enzyme-linked immunosorbent assay), indirect ELISA, sandwich ELISA, competitive ELISA, multiplex ELISA, radioimmunoassay (RIA), ELISPOT technologies, and other similar techniques known in the art. Principles of these immunoassay methods are known in the art, for example John R. Crowther, "The ELISA Guidebook", 1st ed., Humana Press 2000, ISBN 0896037282.

By means of further explanation and not limitation, direct ELISA employs a labelled primary antibody to bind to and thereby quantify target antigen in a sample immobilised on a solid support such as a microwell plate. Indirect ELISA uses a non-labelled primary antibody which binds to the target antigen and a secondary labelled antibody that recognises and allows to quantify the antigen-bound primary antibody. In sandwich ELISA the target antigen is captured from a sample using an immobilised 'capture' antibody which binds to one antigenic site within the antigen, and subsequent to removal of non-bound analytes the so-captured antigen is detected using a 'detection' antibody which binds to another antigenic site within said antigen, where the detection antibody may be directly labelled or indirectly detectable as above. Competitive ELISA uses a labelled 'competitor' that may either be the primary antibody or the target antigen. In an example, non-labelled immobilised primary antibody is incubated with a sample, this reaction is allowed to reach equilibrium, and then labelled target antigen is added. The latter will bind to the primary antibody wherever its binding sites are not yet occupied by non-labelled target antigen from the sample. Thus, the detected amount of bound labelled antigen inversely correlates with the amount of non-labelled antigen in the sample. Multiplex ELISA allows simultaneous detection of two or more analytes within a single compartment (e.g., microplate well) usually at a plurality of array addresses (see, for example, Nielsen & Geierstanger 2004. J Immunol Methods 290: 107-20 and Ling et al. 2007. Expert Rev Mol Diagn 7: 87-98 for further guidance). As appreciated, labelling in ELISA technologies is usually by enzyme (such as, e.g., horse-radish peroxidase) conjugation and the end-point is typically colourimetric, chemiluminescent or fluorescent, magnetic, piezo electric, pyroelectric and other.

Radioimmunoassay (RIA) is a competition-based technique and involves mixing known quantities of radioactively-labelled (e.g., 125I- or 131I-labelled) target antigen with antibody to said antigen, then adding non-labelled or 'cold' antigen from a sample and measuring the amount of labelled antigen displaced (see, e.g., "An Introduction to Radioimmunoassay and Related Techniques", by Chard T, ed., Elsevier Science 1995, ISBN 0444821198 for guidance).

Generally, any mass spectrometric (MS) techniques that can obtain precise information on the mass of peptides, and preferably also on fragmentation and/or (partial) amino acid sequence of selected peptides (e.g., in tandem mass spectrometry, MS/MS; or in post source decay, TOF MS), are useful herein. Suitable peptide MS and MS/MS techniques and systems are well-known per se (see, e.g., Methods in Molecular Biology, vol. 146: "Mass Spectrometry of Proteins and Peptides", by Chapman, ed., Humana Press 2000, ISBN 089603609x; Biemann 1990. Methods Enzymol 193: 455-79; or Methods in Enzymology, vol. 402: "Biological Mass Spectrometry", by Burlingame, ed., Academic Press 2005, ISBN 9780121828073) and may be used herein. MS arrangements, instruments and systems suitable for biomarker peptide analysis may include, without limitation, matrix-assisted laser desorption/ionisation time-of-flight (MALDI-TOF) MS; MALDI-TOF post-source-decay (PSD); MALDI-TOF/TOF; surface-enhanced laser desorption/ionization time-of-flight mass spectrometry (SELDI-TOF) MS; electrospray ionization mass spectrometry (ESI-MS); ESI-MS/MS; ESI-MS/(MS)n (n is an integer greater than zero); ESI 3D or linear (2D) ion trap MS; ESI triple quadrupole MS; ESI quadrupole orthogonal TOF (Q-TOF); ESI Fourier transform MS systems; desorption/ionization on silicon (DIOS); secondary ion mass spectrometry (SIMS); atmospheric pressure chemical ionization mass spectrometry (APCI-MS); APCI-MS/MS; APCI- (MS)n; atmospheric pressure photoionization mass spectrometry (APPI-MS); APPI-MS/MS; and APPI- (MS)n. Peptide ion fragmentation in tandem MS (MS/MS) arrangements may be achieved using manners established in the art, such as, e.g., collision induced dissociation (CID). Detection and quantification of biomarkers by mass spectrometry may involve multiple reaction monitoring (MRM), such as described among others by Kuhn et al. 2004 (Proteomics 4: 1175-86). MS peptide analysis methods may be advantageously combined with upstream peptide or protein separation or fractionation methods, such as for example with the chromatographic and other methods described herein below.

Chromatography can also be used for measuring biomarkers. As used herein, the term "chromatography" encompasses methods for separating chemical substances, referred to as such and vastly available in the art. In a preferred approach, chromatography refers to a process in which a mixture of chemical substances (analytes) carried by a moving stream of liquid or gas ("mobile phase") is separated into components as a result of differential distribution of the analytes, as they flow around or over a stationary liquid or solid phase ("stationary phase"), between said mobile phase and said stationary phase. The stationary phase may be usually a finely divided solid, a sheet of filter material, or a thin film of a liquid on the surface of a solid, or the like. Chromatography is also widely applicable for the separation of chemical compounds of biological origin, such as, e.g., amino acids, proteins, fragments of proteins or peptides, etc.

Chromatography as used herein may be preferably columnar (i.e., wherein the stationary phase is deposited or packed in a column), preferably liquid chromatography, and yet more preferably HPLC. While particulars of chromatography are well known in the art, for further guidance see, e.g., Meyer M., 1998, ISBN: 047198373X, and "Practical HPLC Methodology and Applications", Bidlingmeyer, B. A., John Wiley & Sons Inc., 1993. Exemplary types of chromatography include, without limitation, high-performance liquid chromatography (HPLC), normal phase HPLC (NP-HPLC), reversed phase HPLC (RP-HPLC), ion exchange chromatography (IEC), such as cation or anion exchange chromatography, hydrophilic interaction chromatography (HILIC), hydrophobic interaction chromatography (HIC), size exclusion chromatography (SEC) including gel filtration chromatography or gel permeation chromatography, chromatofocusing, affinity chromatography such as immuno-affinity, immobilised metal affinity chromatography, and the like. Chromatography, including single-, two- or more-dimensional chromatography, may be used as a peptide fractionation method in conjunction with a further peptide analysis method, such as for example, with a downstream mass spectrometry analysis as described elsewhere in this specification.

Further peptide or polypeptide separation, identification or quantification methods may be used, optionally in conjunction with any of the above described analysis methods, for measuring biomarkers in the present disclosure. Such methods include, without limitation, chemical extraction partitioning, isoelectric focusing (IEF) including capillary isoelectric focusing (CIEF), capillary isotachophoresis (CITP), capillary electrochromatography (CEC), and the like, one-dimensional polyacrylamide gel electrophoresis (PAGE), two-dimensional polyacrylamide gel electrophoresis (2D-PAGE), capillary gel electrophoresis (CGE), capillary zone electrophoresis (CZE), micellar electrokinetic chromatography (MEKC), free flow electrophoresis (FFE), etc.

The various aspects and embodiments taught herein may further rely on comparing the quantity of Quiescin Q6 measured in samples with reference value(s) of the quantity of said Quiescin Q6, wherein said reference value(s) represent known predictions, diagnoses and/or prognoses of diseases and disorders linked to impaired beta cell activity, such as those defined in any one of the embodiments of the invention as defined herein.

For example, distinct reference values may represent the prediction of a risk (e.g., an abnormally elevated risk) of having a given disease or condition as taught herein vs. the prediction of no or normal risk of having said disease or condition. In another example, distinct reference values may represent predictions of differing degrees of risk of having such disease or condition.

In a further example, distinct reference values can represent the diagnosis of a given disease or condition as taught herein vs. the diagnosis of no such disease or condition (such as, e.g., the diagnosis of healthy, or recovered from said disease or condition, etc.). In another example, distinct reference values may represent the diagnosis of such disease or condition of varying severity.

In yet another example, distinct reference values may represent a good prognosis for a given disease or condition as taught herein vs. a poor prognosis for said disease or condition. In a further example, distinct reference values may represent varyingly favourable or unfavourable prognoses for such disease or condition.

Such comparison may generally include any means to determine the presence or absence of at least one difference and optionally of the size of such different between values or profiles being compared. A comparison may include a visual inspection, an arithmetical or statistical comparison of measurements. Such statistical comparisons include, but are not limited to, applying a rule. If the values or biomarker profiles comprise at least one standard, the comparison to determine a difference in said values or biomarker profiles may also include measurements of these standards, such that measurements of the biomarker are correlated to measurements of the internal standards.

Reference values for the quantity of any one or more biomarkers may be established according to known procedures previously employed for other biomarkers.

For example, a reference value of the quantity of any one or more biomarkers for a particular diagnosis, prediction and/or prognosis of given disease or condition as taught herein may be established by determining the quantity of said one or more biomarkers in sample(s) from one individual or from a population of individuals characterised by said particular diagnosis, prediction and/or prognosis of said disease or condition (i.e., for whom said diagnosis, prediction and/or prognosis of the disease or condition holds true). Such population may comprise without limitation > 2, > 10, > 100, or even several hundreds or more individuals.

Hence, by means of an illustrative example, reference values of the quantity of Quiescin Q6 for the diagnoses of any one of the disorders linked to impaired beta cell activity or beta cell mass such as for example T1 D, T2D, pancreatitis, pancreatic cancer or tumour formation vs. no such disease or condition may be established by determining the quantity of Quiescin Q6 in sample(s) from one individual or from a population of individuals diagnosed (e.g., based on other adequately conclusive means, such as, for example, clinical signs and symptoms, imaging, ECG, etc.) as, respectively, having or not having said disease or condition.

In an embodiment, reference value(s) as intended herein may convey absolute quantities of Quiescin Q6. In another embodiment, the quantity of Quiescin Q6 in a sample from a tested subject may be determined directly relative to the reference value (e.g., in terms of increase or decrease, or fold-increase or fold-decrease). Advantageously, this may allow to compare the quantity of Quiescin Q6 in the sample from the subject with the reference value (in other words to measure the relative quantity of Quiescin Q6 in the sample from the subject vis-à-vis the reference value) without the need to first determine the respective absolute quantities of Quiescin Q6.

The expression level or presence of Quiescin Q6 in a sample of a patient may sometimes fluctuate, i.e. increase or decrease significantly without change (appearance of, worsening or improving of) symptoms. In such an event, the marker change precedes the change in symptoms and becomes a more sensitive measure than symptom change. Therapeutic intervention can be initiated earlier and be more effective than waiting for deteriorating symptoms. Early intervention at a more benign status may be carried out safely at home, which is a major improvement from treating seriously deteriorated patients in the emergency room.

Measuring the level of Quiescin Q6 of the same patient at different time points can in such a case thus enable the continuous monitoring of the status of the patient and can lead to prediction of worsening or improvement of the patient's condition with regard to diseases and disorders linked to impaired beta cell activity. A home or clinical test kit or device as indicated herein can be used for this continuous monitoring. One or more reference values or ranges of Quiescin Q6 or levels of beta cell activity for such a test can e.g. be determined beforehand or during the monitoring process over a certain period of time in said subject. Alternatively, these reference values or ranges can be established through data sets of several patients with highly similar disease phenotypes, e.g. from healthy subjects or subjects not having the disease or condition of interest. A sudden deviation of the levels of any one or more biomarkers from said reference value or range can predict the worsening of the condition of the patient (e.g. at home or in the clinic) before the (often severe) symptoms actually can be felt or observed.

Also disclosed is thus a method or algorithm for determining a significant change in the level of Quiescin Q6 as taught herein in a certain patient, which is indicative for change (worsening or improving) in clinical status. In addition, the invention allows establishing the diagnosis that the subject is recovering or has recovered from impaired, abnormal, or aberrant beta cell activity linked to a condition selected from the group consisting of: pre-onset type 1 diabetes mellitus (T1 D), pre-clinical T1 D, early onset T1 D, emerging T1 D; pancreatitis such as acute pancreatitis, alcohol induced acute pancreatitis, infectious acute pancreatitis, chronic pancreatitis, alcohol induced chronic pancreatitis, or biliar duct obstruction pancreatitis.

Alternatively, said condition linked to impaired, abnormal, or aberrant beta cell activity is selected from the group consisting of: insulin resistance, exhaustion of beta cells, emerging or developing T2D.

In a further embodiment said condition linked to impaired, abnormal, or aberrant beta cell activity is pancreatic cancer or tumour formation.

In an embodiment the present methods may include a step of establishing such reference value(s). In an embodiment, the present kits and devices may include means for establishing a reference value of the quantity of Quiescin Q6 for a particular diagnosis, prediction and/or prognosis of a condition linked to impaired or aberrant beta cell activity selected from the group consisting of: pre-onset type 1 diabetes mellitus (T1D), pre-clinical T1D, early onset T1D, emerging T1D; pancreatitis such as acute pancreatitis, alcohol induced acute pancreatitis, infectious acute pancreatitis, chronic pancreatitis, alcohol induced chronic pancreatitis, or biliar duct obstruction pancreatitis. Such means may for example comprise one or more samples (e.g., separate or pooled samples) from one or more individuals characterised by said particular diagnosis, prediction and/or prognosis of said disease or condition.

Alternatively, said condition linked to impaired, abnormal, or aberrant beta cell activity is selected from the group consisting of: insulin resistance, exhaustion of beta cells, emerging or developing T2D.

In a further embodiment said condition linked to impaired or aberrant beta cell activity is pancreatic cancer or tumour formation.

The various aspects and embodiments taught herein may further entail finding a deviation or no deviation between the quantity of Quiescin Q6 measured in a sample from a subject and a given reference value.

A "deviation" of a first value from a second value may generally encompass any direction (e.g., increase: first value > second value; or decrease: first value < second value) and any extent of alteration.

For example, a deviation may encompass a decrease in a first value by, without limitation, at least about 10% (about 0.9-fold or less), or by at least about 20% (about 0.8-fold or less), or by at least about 30% (about 0.7-fold or less), or by at least about 40% (about 0.6-fold or less), or by at least about 50% (about 0.5-fold or less), or by at least about 60% (about 0.4-fold or less), or by at least about 70% (about 0.3-fold or less), or by at least about 80% (about 0.2-fold or less), or by at least about 90% (about 0.1-fold or less), relative to a second value with which a comparison is being made.

For example, a deviation may encompass an increase of a first value by, without limitation, at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 150% (about 2.5-fold or more), or by at least about 200% (about 3-fold or more), or by at least about 500% (about 6-fold or more), or by at least about 700% (about 8-fold or more), or like, relative to a second value with which a comparison is being made. Preferably, a deviation may refer to a statistically significant observed alteration. For example, a deviation may refer to an observed alteration which falls outside of error margins of reference values in a given population (as expressed, for example, by standard deviation or standard error, or by a predetermined multiple thereof, e.g., ±1xSD or ±2xSD, or ±1xSE or ±2xSE). Deviation may also refer to a value falling outside of a reference range defined by values in a given population (for example, outside of a range which comprises ≥40%, ≥ 50%, ≥60%, ≥70%, ≥75% or ≥80% or ≥85% or ≥90% or ≥95% or even ≥100% of values in said population).

In a further embodiment, a deviation may be concluded if an observed alteration is beyond a given threshold or cut-off. Such threshold or cut-off may be selected as generally known in the art to provide for a chosen sensitivity and/or specificity of the diagnosis, prediction and/or prognosis methods, e.g., sensitivity and/or specificity of at least 50%, or at least 60%, or at least 70%, or at least 80%, or at least 85%, or at least 90%, or at least 95%.

For example, in an embodiment, an elevated quantity of Quiescin Q6 in the sample from the subject - preferably at least about 1.1-fold elevated, or at least about 1.2-fold elevated, more preferably at least about 1.3-fold elevated, even more preferably at least about 1.4-fold elevated, yet more preferably at least about 1.5-fold elevated, such as between about 1.1-fold and 3-fold elevated or between about 1.5-fold and 2-fold elevated - compared to a reference value representing the prediction or diagnosis of no given disease or condition as taught herein or representing a good prognosis for said disease or condition indicates that the subject has or is at risk of having said disease or condition or indicates a poor prognosis for the disease or condition in the subject, or indicates that the subject does not have or is not at risk of having said disease or condition or indicates a good prognosis for the disease or condition in the subject.

When a deviation is found between the quantity of Quiescin Q6 in a sample from a subject and a reference value representing a certain diagnosis, prediction and/or prognosis of a given disease or condition as taught herein, said deviation is indicative of or may be attributed to the conclusion that the diagnosis, prediction and/or prognosis of said disease or condition in said subject is different from that represented by the reference value.

When no deviation is found between the quantity of Quiescin Q6 in a sample from a subject and a reference value representing a certain diagnosis, prediction and/or prognosis of a given disease or condition as taught herein, the absence of such deviation is indicative of or may be attributed to the conclusion that the diagnosis, prediction and/or prognosis of said disease or condition in said subject is substantially the same as that represented by the reference value.

The above considerations apply analogously to biomarker profiles.

When two or more different biomarkers are determined in a subject, their respective presence, absence and/or quantity may be together represented as a biomarker profile, the values for each measured biomarker making a part of said profile. As used herein, the term "profile" includes any set of data that represents the distinctive features or characteristics associated with a condition of interest, such as with a particular diagnosis, prediction and/or prognosis of a given disease or condition as taught herein. The term generally encompasses inter alia nucleic acid profiles, such as for example genotypic profiles (sets of genotypic data that represents the genotype of one or more genes associated with a condition of interest), gene copy number profiles (sets of gene copy number data that represents the amplification or deletion of one or more genes associated with a condition of interest), gene expression profiles (sets of gene expression data that represents the mRNA levels of one or more genes associated with a condition of interest), DNA methylation profiles (sets of methylation data that represents the DNA methylation levels of one or more genes associated with a condition of interest), as well as protein, polypeptide or peptide profiles, such as for example protein expression profiles (sets of protein expression data that represents the levels of one or more proteins associated with a condition of interest), protein activation profiles (sets of data that represents the activation or inactivation of one or more proteins associated with a condition of interest), protein modification profiles (sets of data that represents the modification of one or more proteins associated with a condition of interest), protein cleavage profiles (sets of data that represent the proteolytic cleavage of one or more proteins associated with a condition of interest), as well as any combinations thereof.

Biomarker profiles may be created in a number of ways and may be the combination of measurable biomarkers or aspects of biomarkers using methods such as ratios, or other more complex association methods or algorithms (e.g., rule-based methods). A biomarker profile comprises at least two measurements, where the measurements can correspond to the same or different biomarkers. A biomarker profile may also comprise at least three, four, five, 10, 20, 30 or more measurements. In one embodiment, a biomarker profile comprises hundreds, or even thousands, of measurements.

Hence, for example, distinct reference profiles may represent the prediction of a risk (e.g., an abnormally elevated risk) of having a given disease or condition vs. the prediction of no or normal risk of having said disease or condition. In another example, distinct reference profiles may represent predictions of differing degrees of risk of having said disease or condition.

In a further example, distinct reference profiles can represent the diagnosis of a given disease or condition as taught herein vs. the diagnosis no such disease or condition (such as, e.g., the diagnosis of healthy, recovered from said disease or condition, etc.). In another example, distinct reference profiles may represent the diagnosis of said disease or condition of varying severity.

In a yet another example, distinct reference profiles may represent a good prognosis for a disease or condition as taught herein vs. a poor prognosis for said disease or condition. In a further example, distinct reference profiles may represent varyingly favourable or unfavourable prognoses for such disease or condition.

Reference profiles used herein may be established according to known procedures previously employed for other biomarkers.

For example, a reference profile of the quantity of any two or more biomarkers for a particular diagnosis, prediction and/or prognosis of diseases and disorders linked to impaired or aberrant beta cell activity, such as those selected from the group consisting of: pre-onset type 1 diabetes mellitus (T1D), pre-clinical T1D, early onset T1D, emerging T1D; pancreatitis such as acute pancreatitis, alcohol induced acute pancreatitis, infectious acute pancreatitis, chronic pancreatitis, alcohol induced chronic pancreatitis, or biliar duct obstruction pancreatitis may be established by determining the profile in sample(s) from one individual or from a population of individuals characterised by said particular diagnosis, prediction and/or prognosis of said disease or condition (i.e., for whom said diagnosis, prediction and/or prognosis of said disease or condition holds true). Such population may comprise without limitation ≥ 2, ≥ 10, ≥ 100, or even several hundreds or more individuals.

Hence, by means of an illustrative example, reference profiles for the diagnoses of diseases and disorders linked to impaired beta cell activity, such as those selected from the group consisting of: pre-onset type 1 diabetes mellitus (T1D), pre-clinical T1D, early onset T1D, emerging T1D; pancreatitis such as acute pancreatitis, alcohol induced acute pancreatitis, infectious acute pancreatitis, chronic pancreatitis, alcohol induced chronic pancreatitis, or biliar duct obstruction pancreatitis vs. no such disease or condition may be established by determining the biomarker profiles in sample(s) from one individual or from a population of individuals diagnosed as, respectively, having or not having said disease or condition.

In an embodiment the present methods may include a step of establishing such reference profile(s). In an embodiment, the present kits and devices may include means for establishing a reference profile for a particular diagnosis, prediction and/or prognosis of diseases and disorders linked to impaired beta cell activity, such as those selected from the group consisting of: pre-onset type 1 diabetes mellitus (T1 D), pre-clinical T1 D, early onset T1 D, emerging T1 D; pancreatitis such as acute pancreatitis, alcohol induced acute pancreatitis, infectious acute pancreatitis, chronic pancreatitis, alcohol induced chronic pancreatitis, or biliar duct obstruction pancreatitis. Such means may for example comprise one or more samples (e.g., separate or pooled samples) from one or more individuals characterised by said particular diagnosis, prediction and/or prognosis of said disease or condition.

Further, art-known multi-parameter analyses may be employed mutatis mutandis to determine deviations between groups of values and profiles generated there from (e.g., between sample and reference biomarker profiles).

When a deviation is found between the sample profile and a reference profile representing a certain diagnosis, prediction and/or prognosis of diseases and disorders linked to impaired beta cell activity, such as those selected from the group consisting of: pre-onset type 1 diabetes mellitus (T1D), pre-clinical T1D, early onset T1D, emerging T1D; pancreatitis such as acute pancreatitis, alcohol induced acute pancreatitis, infectious acute pancreatitis, chronic pancreatitis, alcohol induced chronic pancreatitis, or biliar duct obstruction pancreatitis, said deviation is indicative of or may be attributed to the conclusion that the diagnosis, prediction and/or prognosis of said disease or condition in said subject is different from that represented by the reference profile.

Alternatively, said diseases and disorders linked to impaired beta cell activity are selected from the group consisting of: insulin resistance, exhaustion of beta cells, emerging or developing T2D.

In a further embodiment said diseases and disorders linked to impaired beta cell activity are pancreatic cancer or tumour formation.

When no deviation is found between the sample profile and a reference profile representing a certain diagnosis, prediction and/or prognosis of a given disease or condition as taught herein, the absence of such deviation is indicative of or may be attributed to the conclusion that the diagnosis, prediction and/or prognosis of said disease or condition in said subject is substantially the same as that represented by the reference profile.

The present invention further provides kits or devices for the diagnosis, prediction, prognosis and/or monitoring of any one disease or condition as taught herein comprising means for detecting the level of Quiescin Q6 in a blood or serum sample of the patient. In a more preferred embodiment, such a kit or kits of the invention can be used in clinical settings or at home. The kit according to the invention can be used for diagnosing said beta cell-related disease or condition as defined herein, for monitoring the effectiveness of treatment of a subject suffering from said disease or condition with an agent, or for preventive screening of subjects for the occurrence of said disease or condition in said subject.

The kit or device can be in the form of a bed-side device or in an emergency team setting, e.g. as part of the equipment of an ambulance or other moving emergency vehicle or team equipment or as part of a first-aid kit. The diagnostic kit or device can assist a medical practitioner, a first aid helper, or nurse to decide whether the patient under observation is developing a disease or condition as taught herein, after which appropriate action or treatment can be performed.

Typical kits or devices according to the invention comprise the following elements:
a) a means for obtaining a sample from the subject, such as a blood sample,
b) a means or device for measuring the amount of Quiescin Q6 in said sample and visualizing whether the amount of Quiescin Q6 in said sample is below or above a certain threshold level or value, indicating whether the subject is suffering from a given disease or condition as taught herein or not.

In any of the embodiments of the invention, the kits or devices can additionally comprise c) means for communicating directly with a medical practitioner, an emergency department of the hospital or a first aid post, indicating that a person is suffering from said disease or condition or not.

The term "threshold level or value" or "reference value" is used interchangeably as a synonym and is as defined herein. It can also be a range of base-line (e.g. "dry weight") values determined in an individual patient or in a group of patients with highly similar disease conditions.

In said kit of the invention, the means for obtaining a sample from the subject (a) can be any means for obtaining a sample from the subject known in the art. Examples for obtaining e.g. a blood sample are known in the art and could for example be any kind of finger or skin prick or lancet based device, which basically pierces the skin and results in a drop of blood being released from the skin. The way of providing or obtaining the sample is by no means limiting.

Examples are systems comprising specific binding molecules for said one or more markers attached to a solid phase, e.g. lateral flow strips or dipstick devices and the like well known in the art. Other non-limiting examples of such home test devices, systems or kits can be found for example in the following U.S. patents: 6,107,045, 6,974,706, 5,108,889, 6,027,944, 6,482,156, 6,511,814, 5,824,268, 5,726,010, 6,001,658 or U.S. patent applications: 2008/0090305 or 2003/0109067.

In a preferred embodiment, the invention provides a lateral flow device or dipstick. Such dipstick comprises a test strip allowing migration of a sample by capillary flow from one end of the strip where the sample is applied to the other end of such strip where presence of an analyte in said sample is measured.

In another embodiment, the invention provides a device comprising a reagent strip. Such reagent strip comprises one or more test pads which when wetted with the sample, provide a colour change in the presence of an analyte and/or indicate the concentration of the protein in said sample.

The reference value or range can e.g. be determined using the home device in a period wherein the subject is free of a given disease or condition, giving the patient an indication of his base-line level of any one or more markers. Regularly using the home test device will thus enable the subject to notice a sudden change in levels of said one or more markers as compared to the base-line level, which can enable him to contact a medical practitioner.

Alternatively, the reference value can be determined in the subject suffering from a given disease or condition as taught herein, which then indicates his personal "risk level" for any one or more markers, i.e. the level of said one or more markers which indicates he is or will soon be exposed to said disease or condition. This risk level is interesting for monitoring the disease progression or for evaluating the effect of the treatment.

Furthermore, the reference value or level can be established through combined measurement results in subjects with highly similar disease states or phenotypes (e.g. all having no disease or condition as taught herein or having said disease or condition).

The presence and/or concentration of Quiescin Q6 in a sample can be measured by surface plasmon resonance (SPR) using a chip having binding molecule for said one or more markers immobilized thereon, fluorescence resonance energy transfer (FRET), bioluminescence resonance energy transfer (BRET), fluorescence quenching, fluorescence polarization measurement or other means known in the art. Any of the binding assays described can be used to determine the presence and/or concentration of any one or more markers in a sample. To do so, binding molecule for any one or more markers is reacted with a sample, and the concentration of said one or more markers is measured as appropriate for the binding assay being used. To validate and calibrate an assay, control reactions using different concentrations of standard one or more markers and/or binding molecule for said one or more markers can be performed. Where solid phase assays are employed, after incubation, a washing step is performed to remove unbound markers. Bound marker is measured as appropriate for the given label (e.g., scintillation counting, fluorescence, antibody-dye etc.). If a qualitative result is desired, controls and different concentrations may not be necessary. Of course, the roles of said one or more markers and binding molecule may be switched; the skilled person may adapt the method so binding molecule is applied to sample, at various concentrations of sample.

A binding molecule as intended herein is any substance that binds specifically to any one or more markers. Examples of a binding molecule useful according to the present invention, includes, but is not limited to an antibody, a polypeptide, a peptide, a lipid, a carbohydrate, a nucleic acid, peptide-nucleic acid, small molecule, small organic molecule, or other drug candidate. A binding molecule can be natural or synthetic compound, including, for example, synthetic small molecule, compound contained in extracts of animal, plant, bacterial or fungal cells, as well as conditioned medium from such cells. Alternatively, binding molecule can be an engineered protein having binding sites for said one or more markers. According to an aspect of the invention, a binding molecule binds specifically to said one or more markers with an affinity better than 10-6 M. A suitable binding molecule can be determined from its binding with a standard sample of said one or more markers. Methods for determining the binding between binding molecule and said any one or more markers are known in the art. As used herein, the term antibody includes, but is not limited to, polyclonal antibodies, monoclonal antibodies, humanised or chimeric antibodies, engineered antibodies, and biologically functional antibody fragments (e.g. scFv, nanobodies, Fv, etc) sufficient for binding of the antibody fragment to the protein. Such antibody may be commercially available antibody against said one or more markers, such as, for example, a mouse, rat, human or humanised monoclonal antibody.

The specific-binding agents, peptides, polypeptides, proteins, biomarkers etc. in the present kits may be in various forms, e.g., lyophilised, free in solution or immobilised on a solid phase. They may be, e.g., provided in a multi-well plate or as an array or microarray, or they may be packaged separately and/or individual. The may be suitably labelled as taught herein. Said kits may be particularly suitable for performing the assay methods of the invention, such as, e.g., immunoassays, ELISA assays, mass spectrometry assays, and the like.

The term "modulate" generally denotes a qualitative or quantitative alteration, change or variation specifically encompassing both increase (e.g., activation) or decrease (e.g., inhibition), of that which is being modulated. The term encompasses any extent of such modulation.

For example, where modulation effects a determinable or measurable variable, then modulation may encompass an increase in the value of said variable by at least about 10%, e.g., by at least about 20%, preferably by at least about 30%, e.g., by at least about 40%, more preferably by at least about 50%, e.g., by at least about 75%, even more preferably by at least about 100%, e.g., by at least about 150%, 200%, 250%, 300%, 400% or by at least about 500%, compared to a reference situation without said modulation; or modulation may encompass a decrease or reduction in the value of said variable by at least about 10%, e.g., by at least about 20%, by at least about 30%, e.g., by at least about 40%, by at least about 50%, e.g., by at least about 60%, by at least about 70%, e.g., by at least about 80%, by at least about 90%, e.g., by at least about 95%, such as by at least about 96%, 97%, 98%, 99% or even by 100%, compared to a reference situation without said modulation.

Preferably, modulation of the activity and/or level of intended target(s) (e.g., any one or more markers, nucleic acids, peptides, polypeptides or proteins as taught herein) may be specific or selective, i.e., the activity and/or level of intended target(s) may be modulated without substantially altering the activity and/or level of random, unrelated (unintended, undesired) targets.

Reference to the "activity" of a target may generally encompass any one or more aspects of the biological activity of the target, such as without limitation any one or more aspects of its biochemical activity, enzymatic activity, signalling activity and/or structural activity, e.g., within a cell, tissue, organ or an organism.

In the context of therapeutic or prophylactic targeting of a target, the reference to the "level" of a target may preferably encompass the quantity and/or the availability (e.g., availability for performing its biological activity) of the target, e.g., within a cell, tissue, organ or an organism.

For example, the level of a target may be modulated by modulating the target's expression and/or modulating the expressed target. Modulation of the target's expression may be achieved or observed, e.g., at the level of heterogeneous nuclear RNA (hnRNA), precursor mRNA (pre-mRNA), mRNA or cDNA encoding the target. By means of example and not limitation, decreasing the expression of a target may be achieved by methods known in the art, such as, e.g., by transfecting (e.g., by electroporation, lipofection, etc.) or transducing (e.g., using a viral vector) a cell, tissue, organ or organism with an antisense agent, such as, e.g., antisense DNA or RNA oligonucleotide, a construct encoding the antisense agent, or an RNA interference agent, such as siRNA or shRNA, or a ribozyme or vectors encoding such, etc. By means of example and not limitation, increasing the expression of a target may be achieved by methods known in the art, such as, e.g., by transfecting (e.g., by electroporation, lipofection, etc.) or transducing (e.g., using a viral vector) a cell, tissue, organ or organism with a recombinant nucleic acid which encodes said target under the control of regulatory sequences effecting suitable expression level in said cell, tissue, organ or organism. By means of example and not limitation, the level of the target may be modulated via alteration of the formation of the target (such as, e.g., folding, or interactions leading to formation of a complex), and/or the stability (e.g., the propensity of complex constituents to associate to a complex or disassociate from a complex), degradation or cellular localisation, etc. of the target.

The term "antisense" generally refers to a molecule designed to interfere with gene expression and capable of specifically binding to an intended target nucleic acid sequence. Antisense agents typically encompass an oligonucleotide or oligonucleotide analogue capable of specifically hybridising to the target sequence, and may typically comprise, consist essentially of or consist of a nucleic acid sequence that is complementary or substantially complementary to a sequence within genomic DNA, hnRNA, mRNA or cDNA, preferably mRNA or cDNA corresponding to the target nucleic acid. Antisense agents suitable herein may typically be capable of hybridising to their respective target at high stringency conditions, and may hybridise specifically to the target under physiological conditions.

The term "ribozyme" generally refers to a nucleic acid molecule, preferably an oligonucleotide or oligonucleotide analogue, capable of catalytically cleaving a polynucleotide. Preferably, a "ribozyme" may be capable of cleaving mRNA of a given target protein, thereby reducing translation thereof. Exemplary ribozymes contemplated herein include, without limitation, hammer head type ribozymes, ribozymes of the hairpin type, delta type ribozymes, etc. For teaching on ribozymes and design thereof, see, e.g., US 5,354,855, US 5,591,610, Pierce et al. 1998 (Nucleic Acids Res 26: 5093-5101), Lieber et al. 1995 (Mol Cell Biol 15: 540-551), and Benseler et al. 1993 (J Am Chem Soc 115: 8483-8484).

"RNA interference" or "RNAi" technology is routine in the art, and suitable RNAi agents intended herein may include inter alia short interfering nucleic acids (siNA), short interfering RNA (siRNA), double-stranded RNA (dsRNA), micro-RNA (miRNA), and short hairpin RNA (shRNA) molecules as known in the art. For teaching on RNAi molecules and design thereof, see inter alia Elbashir et al. 2001 (Nature 411: 494-501), Reynolds et al. 2004 (Nat Biotechnol 22: 326-30), http://rnaidesigner.invitrogen.com/rnaiexpress, Wang & Mu 2004 (Bioinformatics 20: 1818-20), Yuan et al. 2004 (Nucleic Acids Res 32(Web Server issue): W130-4), by M Sohail 2004 ("Gene Silencing by RNA Interference: Technology and Application", 1st ed., CRC, ISBN 0849321417), U Schepers 2005 ("RNA Interference in Practice: Principles, Basics, and Methods for Gene Silencing in C.elegans, Drosophila, and Mammals", 1st ed., Wiley-VCH, ISBN 3527310207), and DR Engelke & JJ Rossi 2005 ("Methods in Enzymology, Volume 392: RNA Interference", 1st ed., Academic Press, ISBN 0121827976).

In a preferred embodiment of the present invention, increasing Quiescin Q6 expression or activity seems favourable to induce beta cell activity and hence could be beneficial to treat disorders that are characterised by a decreased beta cell mass or activity. Increasing Quiescin Q6 expression or activating Quiscin Q6 in any other way, will therefore be beneficial for the treatment of disorders or conditions linked to reduced beta cell mass or activity such T1 D (beta cell destruction), T2D (beta cell exhaustion) pancreatitis leading to destruction of beta cells or any other type of disorder leading to the destruction of beta cells or to a decrease in their activity.

Increasing Quiescin Q6 expression can be done using recombinant technologies known in the art. Alternatively, Quiescin Q6 protein can be administered to the beta cells of the subject, or the activity of Quiescin Q6 can be increased using modulating agents. Such agents can be easily identified in known biochemical assays, since the activity of Quiescin Q6 is well known, i.e. the formation of disulfide bridges in proteins. Using in vitro screening assays, it can be easily determined whether or not candidate agents increase or decrease the activity of Quiescin Q6, by looking at the rate or amount of disulfide bridge formation in a substrate protein such as e.g. insulin or any artificial substrate.

The term "pharmaceutically acceptable" as used herein is consistent with the art and means compatible with the other ingredients of a pharmaceutical composition and not deleterious to the recipient thereof.

As used herein, "carrier" or "excipient" includes any and all solvents, diluents, buffers (such as, e.g., neutral buffered saline or phosphate buffered saline), solubilisers, colloids, dispersion media, vehicles, fillers, chelating agents (such as, e.g., EDTA or glutathione), amino acids (such as, e.g., glycine), proteins, disintegrants, binders, lubricants, wetting agents, emulsifiers, sweeteners, colourants, flavourings, aromatisers, thickeners, agents for achieving a depot effect, coatings, antifungal agents, preservatives, antioxidants, tonicity controlling agents, absorption delaying agents, and the like. The use of such media and agents for pharmaceutical active substances is well known in the art. Except insofar as any conventional media or agent is incompatible with the active substance, its use in the therapeutic compositions may be contemplated.

The present active substances (e.g. Quiescin Q6 protein or fragments thereof, nucleic acids encoding therefore, or agents modulating either activity or expression of Quiescin Q6) may be used alone or in combination with any therapies known in the art for the disease and conditions as taught herein ("combination therapy"). Combination therapies as contemplated herein may comprise the administration of at least one active substance of the present invention and at least one other pharmaceutically or biologically active ingredient. Said present active substance(s) and said pharmaceutically or biologically active ingredient(s) may be administered in either the same or different pharmaceutical formulation(s), simultaneously or sequentially in any order.

The dosage or amount of the present active substances (e.g. Quiescin Q6 protein or fragments thereof, nucleic acids encoding therefore, or agents modulating either activity or expression of Quiescin Q6) used, optionally in combination with one or more other active compound to be administered, depends on the individual case and is, as is customary, to be adapted to the individual circumstances to achieve an optimum effect. Thus, it depends on the nature and the severity of the disorder to be treated, and also on the sex, age, body weight, general health, diet, mode and time of administration, and individual responsiveness of the human or animal to be treated, on the route of administration, efficacy, metabolic stability and duration of action of the compounds used, on whether the therapy is acute or chronic or prophylactic, or on whether other active compounds are administered in addition to the agent(s) of the invention.

Without limitation, depending on the type and severity of the disease, a typical daily dosage might range from about 1 µg/kg to 100 mg/kg of body weight or more, depending on the factors mentioned above. For repeated administrations over several days or longer, depending on the condition, the treatment is sustained until a desired suppression of disease symptoms occurs. A preferred dosage of the active substance of the invention may be in the range from about 0.05 mg/kg to about 10 mg/kg of body weight. Thus, one or more doses of about 0.5 mg/kg, 2.0 mg/kg, 4.0 mg/kg or 10 mg/kg (or any combination thereof) may be administered to the patient. Such doses may be administered intermittently, e.g., every week or every two or three weeks.

As used herein, a phrase such as "a subject in need of treatment" includes subjects that would benefit from treatment of a given disease or condition as taught herein. Such subjects may include, without limitation, those that have been diagnosed with said condition, those prone to contract or develop said condition and/or those in whom said condition is to be prevented.

The terms "treat" or "treatment" encompass both the therapeutic treatment of an already developed disease or condition, as well as prophylactic or preventative measures, wherein the aim is to prevent or lessen the chances of incidence of an undesired affliction, such as to prevent the chances of contraction and progression of a disease or condition as taught herein. Beneficial or desired clinical results may include, without limitation, alleviation of one or more symptoms or one or more biological markers, diminishment of extent of disease, stabilised (i.e., not worsening) state of disease, delay or slowing of disease progression, amelioration or palliation of the disease state, and the like. "Treatment" can also mean prolonging survival as compared to expected survival if not receiving treatment.

The term "prophylactically effective amount" refers to an amount of an active compound or pharmaceutical agent that inhibits or delays in a subject the onset of a disorder as being sought by a researcher, veterinarian, medical doctor or other clinician. The term "therapeutically effective amount" as used herein, refers to an amount of active compound or pharmaceutical agent that elicits the biological or medicinal response in a subject that is being sought by a researcher, veterinarian, medical doctor or other clinician, which may include inter alia alleviation of the symptoms of the disease or condition being treated. Methods are known in the art for determining therapeutically and prophylactically effective doses for the present compounds.

The expression "beta cell related disorder" or "disease or condition linked to aberrant, impaired, or abnormal beta cell activity" as used herein carries its art-established meaning. By means of further guidance, said term as used herein broadly refers to pathological conditions involving aberrant, abnormal or impaired beta cell activity, due to a change in total pancreatic beta cell number or due to a change in the activity status of beta cells present in the pancreas. Beta cells can be granular and actively secreting insulin, or they can be immature and ungranulated, not yet secreting insulin. The beta cell number can be changed due to proliferation, new maturation of existing progenitors or by destruction of beta cells, Many disorders can cause a change beta cell activity. Non-limiting examples are: Type 1 diabetes mellitus (T1D), autoimmue reaction to beta cells, pancreatitis, such as: acute pancreatitis, alcohol induced acute pancreatitis, infectious acute pancreatitis, chronic pancreatitis, alcohol induced chronic pancreatitis, biliar duct obstruction pancreatitis. All said disorders or syndromes result in the destruction of beta cells or in the inactivation thereof. Pancreatitis is an inflammation of the pancreas that can occur in an acute or a chronic form. The chronic form often involves fibrosis. The most common cause of chronic pancreatitis is excessive alcohol use, while gallstones are the most common cause of acute pancreatitis. Other possible causes include hypertriglyceridemia, hypercalcemia, viral infection (e.g., mumps), trauma (to the abdomen or elsewhere in the body) including post-ERCP (Endoscopic Retrograde Cholangio-pancreatography), vasculitis (inflammation of the small blood vessels within the pancreas), and autoimmune pancreatitis. Pregnancy can also cause pancreatitis, mostly due to hypertriglyceridemia. Pancreas divisum, a common congenital malformation of the pancreas may underlie some cases of recurrent pancreatitis. Pancreatitis is less common in pediatric population. It is worth noting that pancreatic cancer is seldom the cause of pancreatitis. Type 2 diabetes subjects have 2.8 fold higher risk for pancreatitis compared to non diabetic subjects. Acute hepatic porphyrias, including acute intermittent porphyria, hereditary coproporphyria and variegate porphyria, are genetic disorders that can be linked to both acute and chronic pancreatitis. Acute pancreatitis has also occurred with erythropoietic protoporphyria. Conditions that can lead to gut dysmotility may predispose patients to pancreatitis. This includes the inherited neurovisceral porphyrias and related metabolic disorders. Alcohol, hormones and many drugs including statins are known porphyrinogenic agents. Symptoms and signs of pancreatitis are: severe upper abdominal pain, with radiation through to the back, nausea and vomiting. Findings on the physical exam will vary according to the severity of the pancreatitis, and whether or not it is associated with significant internal bleeding. Typically, both the heart and respiratory rates are elevated. Abdominal tenderness is usually found but may be less severe than expected given the patient's degree of abdominal pain. Bowel sounds may be reduced as a reflection of the reflex bowel paralysis (i.e. ileus) that may accompany any abdominal catastrophe. Diagnosis is currently done by looking whether two of the following three features: 1) abdominal pain characteristic of acute pancreatitis, 2) serum amylase and/or lipase ≥3 times the upper limit of normal, and 3) characteristic findings of acute pancreatitis on CT scan are present. Laboratory tests measuring the level of amylase, lipase or both can be used as well, wherein elevated levels of amylase and/or lipase indicate pancreatitis. Serum lipase may be preferred because it remains normal in some non-pancreatic conditions that increase serum amylase including macroamylasemia, parotitis, and some carcinomas. In general, serum lipase is thought to be more sensitive and specific than serum amylase in the diagnosis of acute pancreatitis. Although amylase is widely available and provides acceptable accuracy of diagnosis, where lipase is available it is preferred for the diagnosis of acute pancreatitis. Ultrasound imaging and CT scanning of the abdomen can be used to confirm the diagnosis of pancreatitis. There are several scoring systems known in the art used to help predict the severity of an attack of pancreatitis, such as the Apache II, Glasgow and Ranson criteria. Mild acute pancreatitis is characterized by spotty peripancreatic fat necrosis, which is resolved without inducing significant fibrosis. Severe acute pancreatitis with large confluent areas of peripancreatic necrosis, but little intrapancreatic involvement, leads to an extrapancreatic pseudocyst. Relapse of severe acute pancreatitis with extensive extra- and intrapancreatic foci of necrosis induces perilobular fibrosis and duct distortions. In addition, there may be extrapancreatic pseudocysts. Early stage chronic pancreatitis can evolve into end-stage chronic pancreatitis with severe duct changes, diffuse but still patchy fibrosis and calculi.

Other exemplary stages of pancreatitis are (Reviewed e.g. by Günter Klöppel in Modern Pathology (2007) 20, S113-S131,): autoimmune pancreatitis, paraduodenal pancreatitis, obstructive chronic pancreatitis, chronic pancreatitis, alcoholic pancreatitis, non-alcoholic pancreatitis, hereditary pancreatitis, metabolic pancreatitis (hypercalcemia, hyperlipidemia), autoimmune pancreatitis, idiopathic pancreatitis, tropical pancreatitis, chronic pancreatitis associated with anatomic abnormalities, periampullary duodenal wall cysts, pancreas divisum, post-traumatic pancreatic scars, pancreatic fibrosis in the elderly, cystic fibrosis, pancreatic fibrosis in long-term insulin dependent diabetes mellitus, hemochromatosis, etc..

Pancreatic tumours and pancreatic cancer can also be catalogued as a disease or disorder linked to impaired or aberrant beta cell activity. The most famous pancreatic tumour is "insulinoma", a tumour of the pancreas derived from beta cells and actively secreting insulin. The secretion of insulin by insulinomas is not properly regulated by glucose and the tumours will continue to secrete insulin causing glucose levels to fall further than normal. As a result patients will show symptoms of low blood glucose (hypoglycemia). The diagnosis of an insulinoma is usually made biochemically with low blood glucose, elevated insulin, proinsulin and C-peptide levels and confirmed by localising the tumour with medical imaging or angiography. The definitive treatment is surgery, prognosis is generally good. Pancreatic cancer on the other hand can have many forms. The prognosis is poor, with fewer than 5% of those diagnosed still alive five years after diagnosis. Most exocrine pancreatic cancers are adenocarcinomas (M8140/3). The remaining cancers include adenosquamous carcinomas, signet ring cell carcinomas, hepatoid carcinomas, colloid carcinomas, undifferentiated carcinomas, and undifferentiated carcinomas with osteoclast-like giant cells. Exocrine pancreatic tumours are far more common than pancreatic endocrine tumours such as insulinomas and other pancreatic islet cell tumours. The latter however will most often result in an increased beta cell mass or activity, while in general exocrine pancreatic cancers or tumours will ultimately result in destruction of beta cell mass and activity, which are overgrown or destroyed due to inflammatory responses in reaction to tumour growth.

Type 1 diabetes mellitus (T1 D) is another major cause of impaired beta cell activity, since in T1 D, the immune system of the subject attacks and destroys its own beta cells. T1 D is an autoimmune disorder, which can be triggered by both genetic predisposition and numerous environmental factors such as: viral or bacterial infection or other allergens in e.g. cow milk or wheat or use of chemicals and drugs. All this causes an initial inflammation in the pancreas. Due to this, part of the beta cells can be destroyed, which will trigger them to proliferate, generating even more antigens, which can then cause an auto-immune reaction destroying even more beta cells. This chain reaction cannot be followed or predicted by any means at the moment, but it is established that clinical manifestation of T1D reflects the consequence of an underlying, sustained autoimmune process. For instance, autoantibodies against islet antigens are detected before the clinical onset of T1D. This suggests that a sequence of inciting events precedes the hyperglycemia for at least months, but most likely several years. This wide gap between initiation and detection of ongoing diabetogenic events poses a cardinal problem in the search for causative environmental triggers (cf. Van Belle et al., 2011 for review). This implies that a simple analysis of the Quiescin Q6 blood level in a subject as envisaged by the present invention, during e.g. a standard blood analysis could help in predicting the onset of T1 D and help in reacting with the appropriate treatment, well before symptoms are actually seen. Follow up or monitoring of patients with a high risk (e.g. predisposed) of developing T1D can be done daily, weekly or monthly, based e.g. on the clinical history and diet of the patient. For all subject, a yearly check up including a blood analysis could include a Quiescin Q6 analysis in order to indicate the risk of development of diabetes, even before clinical symptoms are apparent. In this respect, it is important to note that both pancreatitis and pancreas tumours or cancer can eventually also lead to T1D, since they also imply the occurrence of an inflammatory reaction in the pancreas. characterized by recurrent or persistent hyperglycemia, and is diagnosed by demonstrating any one of the following:
- Fasting plasma glucose level at or above 7.0 mmol/L (126 mg/dL),
- Plasma glucose at or above 11.1 mmol/L (200 mg/dL) two hours after a 75 g oral glucose load as in a glucose tolerance test,
- Symptoms of hyperglycemia and casual plasma glucose at or above 11.1 mmol/L (200 mg/dL).
(cf. World Health Organisation: Department of Noncommunicable Disease Surveillance (1999). "Definition, Diagnosis and Classification of Diabetes Mellitus and its Complications")

In addition, the appearance of diabetes-related autoantibodies has been shown to be able to predict the appearance of diabetes type 1 before hyperglycemia arises: islet cell autoantibodies, insulin autoantibodies, autoantibodies targeting the 65 kDa isoform of glutamic acid decarboxylase (GAD) and autoantibodies tergeting the phosphatase-related IA-2 molecule are known to be important.

Although T1D is not actually preventable, promising therapies are slowly emerging, and it has been suggested that, in the future, diabetes type 1 may be prevented at the latent autoimmune stage, probably by a combination therapy of several methods (Bluestone et al., 2010, Nature 464 (7293): 1293). Early detection of T1D is of course of great importance herein and the present application provides an easy tool therefore. Cyclosporine A, an immunosuppressive agent, can be used to halt destruction of beta cells. Also anti-CD3 antibodies, including teplizumab and otelixizumab, have evidence of preserving insulin production (as evidenced by sustained C-peptide production) in newly diagnosed type 1 diabetes patients. An anti-CD20 antibody, rituximab, inhibits B cells and has been shown to provoke C-peptide responses three months after diagnosis of type 1 diabetes, but long-term effects of this have not been reported. Furthermore, injections with a vaccine containing GAD65, an autoantigen involved in type 1 diabetes, has in clinical trials delayed the destruction of beta cells when treated within six months of diagnosis (Bluestone et al., 2010, Nature 464 (7293): 1293).

T1D is usually treated with insulin replacement therapy. This can be done using subcutaneous injection or with an insulin pump, along with attention to dietary management (especially carbohydrates), and monitoring of blood glucose levels using glucose meters which can be simply operated by the patient himself. Also the insulin injections are often performed by the patients themselves. Untreated type 1 diabetes commonly leads to coma, often from diabetic ketoacidosis, which can be fatal. In some cases pancreas transplantation or islet (beta) cell grafting is used as a form of treatment to restore proper glucose regulation. This is however a very severe intervention, both on the level of surgery and the accompanying immunosuppression in order to prevent rejection of the transplanted tissue. Long time monitoring and follow up of successful transplantation is required and the present invention also provides the tools for aiding in this monitoring by showing that Quiescin Q6 levels in blood can be used to measure beta cell activity. A restored activity (to normal reference levels) or a maintained or reduced activity after transplantation can be followed by measuring Quiescin Q6 in blood. Islet cell transplantation. Beta cells can be derived from a pancreatic transplant or from stem cells.

T1D can have a long disease development and can start long before clinical signs become apparent. Several stages are defined: No- T1D, with normal beta cell function/mass, pre-onset T1D, with emerging auto-antibody titers to beta cells, due to e.g. inflammatory reactions; early onset T1D with initial beta cell destruction; first clinical signs such as disturbed Oral Glucose Tolerance Test; newly onset T1D, T1D being treated with insulin, resulting in the so-called honeymoon period of amelioration of blood glucose homeostasis due to recovery of remaining beta cells; after said honeymoon period, wherein the beta cell destruction is continued.

The honeymoon period for patients with T1D is the period after the disease is diagnosed and insulin treatment is started. During this period some of the insulin-producing beta cells have not been destroyed. The insulin treatment will in many cases allow the beta cells to recover and produce some amount of insulin. As a result the doses of injected insulin can be decreased and blood sugar control is improved. The honeymoon period does not occur in all patients and normally does not last for ever.

Type 2 diabetes (T2D) is mostly caused by insulin resistance and can eventually result in beta cell exhaustion, leading also to /or as a sign off beta cell destruction. T2D is a condition in which body cells fail to use insulin properly, sometimes combined with an absolute insulin deficiency. T2D is also known as non-insulin-dependent diabetes mellitus (NIDDM) or adult-onset diabetes. Insulin resistance is the defective responsiveness of body tissues to insulin and is believed to involve the insulin receptor, although the specific defects are yet unknown. In the early stage of T2D, the predominant abnormality is reduced insulin sensitivity. At this stage hyperglycemia can be reversed by a variety of measures and medications known in the art. T2D develops from insulin resistance, meaning that the normally secreted dose of insulin is no longer sufficient to control blood glucose levels. In a reaction to this process, beta cells are forced to produce more insulin, or are triggered to proliferate and/or granulate, producing more insulin. This overproduction of insulin or over activity of beta cells can then lead to beta cell exhaustion, leading to destruction of the beta cell population. This process can thus be followed easily be measuring the beta cell activity, for which the Quiescin Q6 protein is a good marker in blood. Insulin resistance syndrome or simply metabolic syndrome or metabolic syndrome X is one of the pathophysiological conditions that cause or underlie T2D and can be linked to both genetic predisposition and many environmental factors such as diet, stress, overweight, aging, certain infections, coronary heart disease etc.

As used herein, the terms "Quiescin Q6", "QSOX1" and "Sulfhydryl oxidase 1" are synonymous and refer to proteins and polypeptides commonly known under these designations in the art. The terms encompass such proteins and polypeptides of any organism where found, and particularly of animals, preferably vertebrates, more preferably mammals, including humans and non-human mammals, even more preferably of humans. The terms particularly encompass such proteins and polypeptides with a native sequence, *i.e.*, ones of which the primary sequence is the same as that of Quiescin Q6 found in or derived from nature. A skilled person understands that native sequences of Quiescin Q6 may differ between different species due to genetic divergence between such species. Moreover, the native sequences of Quiescin Q6 may differ between or within different individuals of the same species due to normal genetic diversity (variation) within a given species. Also, the native sequences of Quiescin Q6 may differ between or even within different individuals of the same species due to post-transcriptional or post-translational modifications. Accordingly, all Quiescin Q6 sequences found in or derived from nature are considered "native". The terms encompass Quiescin Q6 proteins and polypeptides when forming a part of a living organism, organ, tissue or cell, when forming a part of a biological sample, as well as when at least partly isolated from such sources. The terms also encompass proteins and polypeptides when produced by recombinant or synthetic means.

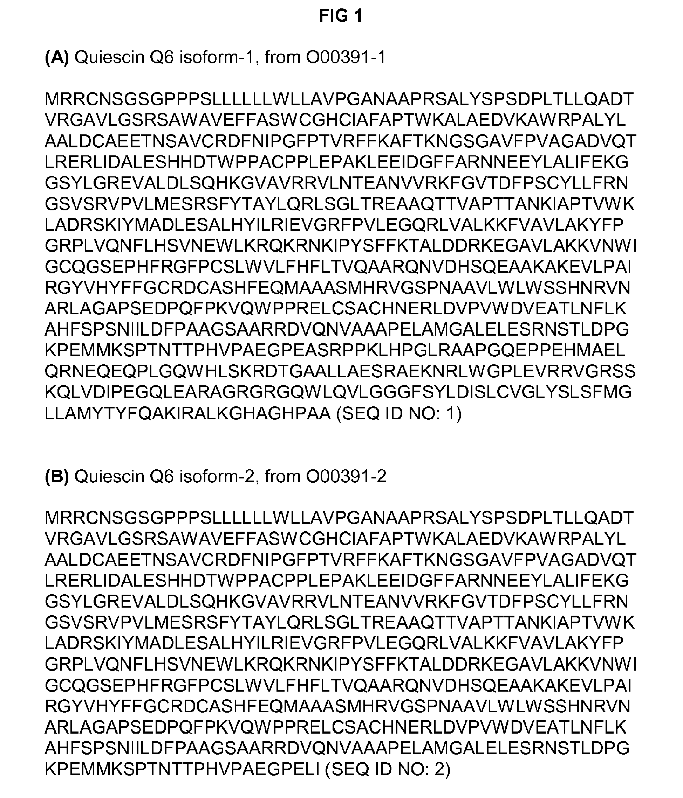

Exemplary Quiescin Q6 includes, without limitation, human Quiescin Q6 having primary amino acid sequence as annotated under Uniprot/Swissprot (http://www.expasy.org/) accession number 000391 (entry version 69 revised on January 20, 2009; sequence version 3 created on June 1, 2001), including isoform 1 (acc. no. 000391-1) and isoform 2 (000391-2) generated due to alternative splicing. The sequence of said isoforms 1 and 2 of Quiescin Q6 is shown in Fig. 1A (SEQ ID NO: 1) and Fig. 1B (SEQ ID NO: 2), respectively. Figure 2 illustrates the differences in the C-terminal region between said isoforms 1 and 2. A skilled person can also appreciate that said sequences are of precursor of Quiescin Q6 and may include parts which are processed away from mature Quiescin Q6. For example, with reference to the isoform 1 sequence, the Uniprot/Swissprot entry specifies a signal peptide composed of amino acids 1-29. Exemplary human Quiescin Q6 has been also described *inter alia* by Coppock et al. 1998 (Genomics 54: 460-468).

The reference herein to Quiescin Q6 may also encompass fragments of Quiescin Q6. Hence, the reference herein to measuring Quiescin Q6, or to measuring the quantity of Quiescin Q6, may encompass measuring the Quiescin Q6 protein or polypeptide (such as, e.g., measuring the mature isoform 1 and/or isoform 2 of Quiescin Q6) and/or measuring one or more fragments of Quiescin Q6. For example, Quiescin Q6 and/or one or more fragments thereof may be measured collectively, such that the measured quantity corresponds to the sum amounts of the collectively measured species. In another example, Quiescin Q6 and/or one or more fragments thereof may be measured each individually.

Using immunohistochemistry, Quiescin Q6 expression has been reported in numerous tissues, mainly of the endocrinal, gland and *villi* tissue types (cf. e.g. information in the public domain: http://thorpelab.chem.udel.edu/index.php/qsox-ology). Amongst the reported tissues, also pancreatic, beta cells, show Quiescin Q6 expression. Interestingly, also insulinoma's, insulin producing tumours originating from beta cells show high expression of Quiescin Q6.

Q6 is known to introduce disulphide bridges in proteins and one of its candidate target proteins is insulin, which indeed contains 3 S-S bridges. Without wanting to be bound by any theory, we assume the activity or involvement of Quiescin Q6 in the generation of these disulphide bridges in insulin.

In the beta cells within islets of Langerhans of the pancreas, insulin is originally produced as a single molecule, called pre-pro-insulin, composed of 110 amino acids. After this has passed through the endoplasmic reticulum, 24 amino acids ("the signal peptide") are removed by enzyme action from one end of the chain, resulting in pro-insulin, which folds and bonds to give the molecule much of the final structure. This passes into vesicles budded off from the Golgi body. Here a middle section ("the C chain") of 33 amino-acids is removed by the action of the enzymes pro-hormone convertase 1 and 2, converting it into the final structure with 2 chains, A and B, and 2 amino acids are then removed by another enzyme carboxypeptidase E. The final three-dimensional structure of insulin is then further stabilised by disulphide bridges. These form between thiol groups (-SH) on cysteine residues (CYS above). There are 6 cysteines, so 3 disulphide bridges are formed: 2 between the A and B chains, and one within the A chain.

Indeed, the present invention clearly shows that the expression of Quiescin Q6 is elevated in blood samples of pancreatic cancer patients and in blood samples of patients suffering from chronic pancreatitis. Although the expression of Quiescin Q6 in serum of pancreatic cancer patients was already confirmed by Antwi and coworkers (J. Prot Res, 2009, 8:4722-4731) this report is silent about expression in inflamed or infected pancreas. In an additional paper (Altirriba et al., 2009, BMC Genomics 10:406). mRNA expression data (online) indeed shows Quiescin Q6 expression in pancreas tissue of diabetes rat model with and without treatment with tungstate, which increases beta cell mass. Quiescin Q6 expression in the pancreas is lower in diabetic rats (having lower beta cell-content), and is restored following treatment restoring beta cell function.

Remarkably, the present invention shows that the Quiescin Q6 expression in serum of patients largely reflects the Quiescin Q6 expression in pancreatic tissue. The Quiescin Q6 level in blood is moreover correlated to beta cell functioning in general, regardless of the type of beta cell related disorder or condition. This enables a much less invasive method for diagnosis, prognosis, prediction and/or monitoring of beta cell functioning or activity, influenced by a whole range of conditions, such as: pre-onset type 1 diabetes mellitus (T1D), pre-clinical T1D, early onset T1D, emerging T1D; pancreatitis such as acute pancreatitis, alcohol induced acute pancreatitis, infectious acute pancreatitis, chronic pancreatitis, alcohol induced chronic pancreatitis, or biliar duct obstruction pancreatitis; or alternatively of insulin resistance, exhaustion of beta cells, emerging or developing T2D; or alternatively of pancreatic cancer or tumour formation.

Using e.g. the NOD mouse model of type 1 diabetes, the inventors can show that Quiescin Q6 expression is indeed linked to beta cell functioning and that it can be used to predict, diagnose, or for prognosis of beta cell dysfunction. In addition, the expression of Quiescin Q6 can be used to monitor beta cell disorder treatment. More specifically, increased quiescin Q6 presence in a blood sample (whole blood, serum or plasma) of a subject is indicative of increased beta cell activity, e.g. reflected by increased insulin production. This can be due to beta cell function regeneration, reactivation, proliferation, maturation (or granulation) or the like.

In addition, patient samples of T1D patients can be analysed before and during and after treatment, in order to monitor progression of disease treatment.

The use of Quiescin Q6 as a marker for beta cell activity as defined in the present invention can be used for patient selection and timing of therapy.

This can e.g. be done by selecting within the group of genetic susceptible subjects (cf. for review Van Belle et al., 2011), those subjects who will indeed progress towards beta cell destruction/dysfunction and those who will not. It should be noted that many of the genetic susceptible subjects do not progress towards diabetes.

Additionally, the timing of the intervention can be decided upon more easily by using the Quiescin Q6 marker of the invention to measure beta cell activity. It is important that sufficient beta cell mass is preserved to be successful with certain therapies. Early intervention usually coincide with a smaller loss of β-cell mass. In addition, the most appropriate intervention can be chosen based on the knowledge that stimulation of β-cell proliferation is only successful when sufficient β-cell mass is left and that in patients with low remaining β-cell mass transplantation or stem-cell therapy is a better therapeutic option.

Since diabetes mellitus can be caused by autoimmune reactions, it can be helpful to use immune-modulating therapies in order to prevent disease, i.e. in the form of active killing of beta cells. Knowing from an early stage on that something is going wrong with the beta cell survival or activity level is therefore very important to enable a timely start of the treatment. Also during the treatment, monitoring of the beta cell activity level is of great importance in order to modulate the treatment where necessary. The simple measurement of the Quiescin Q6 levels in blood samples enables such a timely and fast decision making process.

Using Quiescin Q6 in order to measure beta cell activity can also be used to select the most appropriate drugs or monoclonal antibodies for said immunomodulatory treatment. This is especially the case because at different stages of the destruction process, reflected by different degrees of loss of beta cell function and different degrees of decrease in Quiescin Q6 blood levels, different interventions may be needed for obtaining the best results.

The dose of the treatment can be adjusted more appropriately using the Quiescin Q6 level measurement of the invention. A positive feedback on the success of the immunomodulatory intervention might allow for the lowest effective dose, thus enabling fewer side effects. Alternatively, negative feedback might trigger the choice of a more appropriate intervention.

In a further aspect, the present invention can be used for beta cell transplantation surveillance. Measuring the level of Quiescin Q6 in a blood sample of the subject reflects the total beta cell activity and hence an increased presence of Quiescin Q6 after beta cell transplant indicates that the grafted beta cells are indeed active and that hence the transplantation was successful. Monitoring the level of Quiescin Q6 in the blood of the subject over time thus will enable the surveillance of the survival rate of the transplanted beta cells.

In addition, the immune-suppression treatment can be fine-tuned based on the above results of the monitoring or surveillance process measuring Quiescin Q6 in a blood sample of the subject; One can envisage increased immunosuppression therapy, in case of a change in beta cell activity (reflected by changing Quiescin Q6 levels in the blood sample), or maintained or decreased immunosuppression therapy in case the grafting process seems promising, i.e. when the beta cells are actively producing insulin and the Quiescin Q6 levels are in a steady state, or are comparable to those of a healthy subject.

In an additional aspect, the present invention allows the identification of patients with a degree of insulin resistance or enables the assessment of the degree of said insulin resistance. In principle, when more insulin synthesis is needed to preserve a certain glycemic control, the patient might be called to have "insulin resistance". Treating insulin resistance allows to preserve beta cell function longer (years), effectively preventing the evolution towards T2D. Measuring the insulin resistance with a biomarker would greatly improve the practicality and ease of use of the current tests such as: the hyperinsulinemic euglycemic clamp, modified insulin suppression test the HOMA, or the QUICKI tests, which are all quite invasive, discomforting, and sometimes entail health risks. Measuring a single biomarker (Quiescin Q6) level in a blood sample, in order to detect or predict the onset of insulin resistance clearly is advantageous over all the known techniques. This measurement can be combined with other known biomarkers or parameters useful for the measurement of beta cell mass or activity, such as insulin, or pro-insulin production or secretion, blood glucose level, VMAT2 expression, etc..

The above aspects and embodiments are further supported by the following non-limiting examples.

### EXAMPLES

Example 1: MASSterclass targeted protein quantification for early validation of candidate markers derived from discovery.

### MASSTERCLASS experimental setup

MASSterclass assays use targeted tandem mass spectrometry with stable isotope dilution as an end-stage peptide quantification system (also called Multiple Reaction Monitoring (MRM) and Single Reaction Monitoring (SRM)). The targeted peptide is specific (i.e., proteotypic) for the specific protein of interest. i.e., the amount of peptide measured is directly related to the amount of protein in the original sample. To reach the specificity and sensitivity needed for biomarker quantitation in complex samples, peptide fractionations precede the end-stage quantitation step.

A suitable MASSTERCLASS assay may include the following steps:
- Plasma/serum sample
- Depletion of human albumin and IgG (complexity reduction on protein level) using affinity capture with anti-albumin and anti-IgG antibodies using ProteoPrep spin columns (Sigma Aldrich)
- Spiking of known amounts of isotopically labelled peptides. This peptide has the same amino acid sequence as the proteotypic peptide of interest, typically with one isotopically labelled amino acid built in to generate a mass difference. During the entire process, the labelled peptide has identical chemical and chromatographic behaviour as the endogenous peptide, except during the end-stage quantitation step which is based on molecular mass.
- Tryptic digest. The proteins in the depleted serum/plasma sample are digested into peptides using trypsin. This enzyme cleaves proteins C-terminally from lysine and argninine, except when a proline is present C-terminally of the lysine or arginine. Before digestion, proteins are denatured by boiling, which renders the protein molecule more accessible for the trypsin activity during the 16h incubation at 37°C.
- First peptide-based fractionation: Free Flow Electrophoresis (FFE; BD Diagnostic) is a gel-free, fluid separation technique in which charged molecules moving in a continuous laminar flow are separated through an electrical field perpendicular to the flow. The electrical field causes the charged molecules to separate in the pH gradient according to their isoelectric point (pl). Only those fractions containing the monitored peptides are selected for further fractionation and LC-MS/MS analysis. Each peptide of interest elutes from the FFE chamber at a specific fraction number, which is determined during protein assay development using the synthetic peptide homologue. Specific fractions or fraction pools (multiplexing) proceed to the next level of fractionation.
- Second peptide-based fractionation: Phenyl HPLC (XBridge Phenyl; Waters) separates peptides according to hydrophobicity and aromatic nature of amino acids present in the peptide sequence. Orthogonality with the back-end C18 separation is achieved by operating the column at an increased pH value (pH 10). As demonstrated by Gilar et al. 2005, J Sep Sci 28(14): 1694-1703), pH is by far the most drastic parameter to alter peptide selectivity in RP-HPLC. Each peptide of interest elutes from the Phenyl column at a specific retention time, which is determined during protein assay development using the synthetic peptide homologue. The use of an external control system, in which a mixture of 9 standard peptides is separated upfront a batch of sample separations, allows adjusting the fraction collection in order to correct for retention time shifts. The extent of fractionation is dependent on the concentration of the protein in the sample and the complexity of that sample.
- LC-MS/MS based quantitation, including further separation on reversed phase (C18) nanoLC (PepMap C18; Dionex) and MS/MS: tandem mass spectrometry using MRM (4000 QTRAP; ABI)/SRM (Vantage TSQ; Thermo Scientific) mode. The LC column is connected to an electrospray needle connected to the source head of the mass spectrometer. As material elutes from the column, molecules are ionized and enter the mass spectrometer in the gas phase. The peptide that is monitored is specifically selected to pass the first quadrupole (Q1), based on its mass to charge ratio (m/z). The selected peptide is then fragmented in a second quadrupole (Q2) which is used as a collision cell. The resulting fragments then enter the third quadrupole (Q3). Depending on the instrument settings (determined during the assay development phase) only a specific peptide fragment or specific peptide fragments (or so called transitions) are selected for detection.
- The combination of the m/z of the monitored peptide and the m/z of the monitored fragment of this peptide is called a transition. This process can be performed for multiple transitions during one experiment. Both the endogenous peptide (analyte) and its corresponding isotopically labelled synthetic peptide (internal standard) elute at the same retention time, and are measured in the same LC-MS/MS experiment.
- The MASSterclass readout is defined by the ratio between the area under the peak specific for the analyte and the area under the peak specific for the synthetic isotopically labelled analogue (internal standard). MASSterclass readouts are directly related to the original concentration of the protein in the sample. MASSterclass readouts can therefore be compared between different samples and groups of samples.

A typical MASSTERCLASS protocol followed in the present study is given here below:
- 25µL of plasma is subjected to a depletion of human albumin and IgG (ProteoPrep spin columns; Sigma Aldrich) according to the manufacturer's protocol, except that 20mM NH₄HCO₃ was used as the binding/equilibration buffer.
- The depleted sample (225µL) is denatured for 15min at 95°C and immediately cooled on ice
- 500 fmol of the isotopically labelled peptide (custom made 'Heavy AQUA' peptide; Thermo Scientific) is spiked in the sample
- 20µg trypsin is added to the sample and digestion is allowed for 16h at 37°C
- The digested sample was first diluted 1/8 in solvent A (0.1% formic acid) and then 1/20 in the same solvent containing 250 amol/µL of all isotopically labelled peptides (custom made 'Heavy AQUA' peptide; Thermo Scientific) of interest.
- 20µL of the final dilution was separated using reverse-phase NanoLC with on-line MS/MS in MRM/SRM mode:
   - Column: PepMap C18, 75µm I.D. x 25cm L, 100 A pore diameter, 5µm particle size
   - Solvent A: 0.1% formic acid
   - Solvent B: 80% acetonitrile, 0.1 % formic acid
   - Gradient: 30 min; 2%-55% Solvent B
   - MS/MS in MRM mode: method contains the transitions for the analyte as well as for the synthetic, labelled peptide.
   - The used transitions were experimentally determined and selected during protein assay development
   - Each of the transitions of interest was measured for a period starting 3 minutes before and ending 3 minutes after the determined retention time of the peptide of interest, making sure that each peak had at least 15 datapoints.
   - The raw data was analysed and quantified using the LCQuan software (Thermo Scientific): the area under the analyte (= the Quiescin Q6 peptide) peak and under the internal standard (the labelled, synthetic Quiescin Q6 peptide) peak at the same C18 retention time was determined by automatic peak detection. These were cross-checked manually.
   - The MASSterclass readout was defined by the ratio of the analyte peak area and the internal standard peak area

### MASSTERCLASS data analysis

The measured ratios are differential quantitations of peptides. In other words a ratio is the normalised concentration of a peptide. The concentration of a peptide is proportional to the ratio measured in the mass spectrometer.

### Example 2: Screening different disease populations for Quiescin Q6 reveals increased levels in chronic pancreatitis and pancreas cancer

The MASSterclass Quiescin Q6 assay was used to screen patient plasma samples from a variety of diseases including:
- Patients with chronic heart failure, clinically stable at time of sampling
- Patients admitted to ED with systemic inflammatory response syndrome (SIRS)
- Patients admitted to ED with confirmed SEPSIS
- Patients with chronic pancreatitis
- Patients with confirmed stage III or IV pancreatic cancer
- Pregnant women at 22 weeks of gestation pre-destined to develop pre-eclampsia later during the pregnancy
- Healthy pregnant women at 22 weeks of gestation

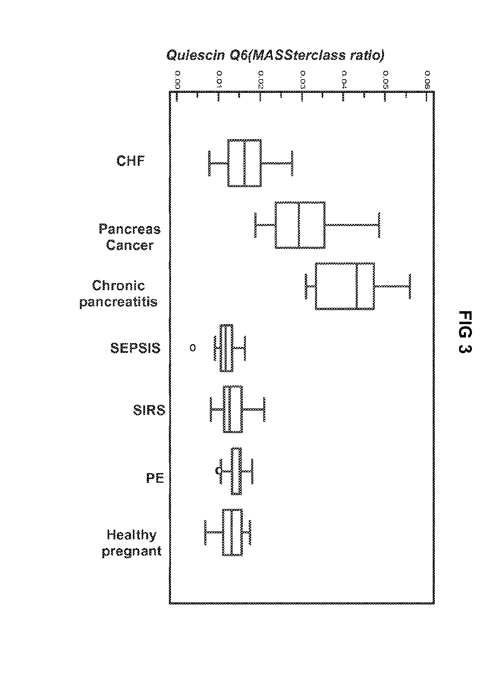

Figure 3 shows box and whisker plots for Quiescin Q6 over the different disease populations. Table 1 summarizes median relative Quiescin Q6 levels and quartiles for the different disease populations. Levels of Quiescin Q6 are clearly elevated in samples from patients with chronic pancreatitis and advanced pancreas cancer. Levels for Quiescin Q6 are on median levels 3.5 fold and 2.3 fold higher in chronic pancreatitis and pancreas cancer respectively compared to SIRS, SEPSIS and healthy pregnant patients.

**Table 1: Median levels and interquartile ranges for Quiescin Q6 as measured by MASSterclass over the different disease populations**

| | ***# patients*** | ***median*** | ***interquartile range*** |
|---|---|---|---|
| **CHF** | 13 | 0.0163 | 0.0124 - 0.0202 |
| **Pancreas Cancer** | 24 | 0.0293 | 0.0237 - 0.0355 |
| **Chronic Pancreatitis** | 12 | 0.0432 | 0.0335 - 0.0474 |
| **SEPSIS** | 15 | 0.0117 | 0.0106 - 0.0133 |
| **SIRS** | 15 | 0.0127 | 0.0114 - 0.0156 |
| **Pyre-eclampsia** | 15 | 0.015 | 0.0133 - 0.0153 |
| **healthy pregnant control** | 15 | 0.0131 | 0.0111 - 0.0156 |

These results clearly show that the expression of Quiescin Q6 is elevated in blood samples of pancreatic cancer patients and in blood samples of patients suffering from chronic pancreatitis. In both conditions, beta cell mass and/or activity is often severely impaired due to inflammatory responses and tissue destruction. Although the expression of Quiescin Q6 in serum of pancreatic cancer patients was already confirmed by Antwi and coworkers (J. Prot Res, 2009, 8:4722-4731) said report is silent about expression in inflamed or infected pancreas. The results provided herein show that Quiescin Q6 is a general marker for indicating problems related to beta cell mass or pancreas functioning, rather than being a specific pancreatic cancer marker.

The present experimental results furthermore show that the Quiescin Q6 expression in serum of patients largely reflects the Quiescin Q6 expression in pancreatic tissue. The Quiescin Q6 level in blood is unexpectedly correlated to beta cell functioning in general, regardless of the type of beta cell related disorder or condition (e.g. whether the patient has pancreatic cancer or pancreatitis). This enables a much less invasive method for diagnosis, prognosis, prediction and/or monitoring of beta cell functioning or activity, influenced by a whole range of conditions, such as: pre-onset type 1 diabetes mellitus (T1D); pre-clinical T1D, early onset T1D, and emerging T1D; insulin resistance, exhaustion of beta cells, emerging or developing T2D, pancreatitis such as acute pancreatitis, alcohol induced acute pancreatitis, infectious acute pancreatitis, chronic pancreatitis, alcohol induced chronic pancreatitis, or biliar duct obstruction pancreatitis, pancreatic cancer or tumour formation.

### Example 3: Quiescin Q6 as a general marker for beta cell (dys)functioning

In this experiment, the level of Quiescin Q6 protein is assessed in subjects suffering from beta cell dysfunctioning, due to type 1 diabetes mellitus (T1D), pancreatitis (acute pancreatitis, alcohol induced acute pancreatitis, infectious acute pancreatitis, chronic pancreatitis, alcohol induced chronic pancreatitis, or biliar duct obstruction pancreatitis), pancreas cancers or tumours (e.g. insulinomas), using either the above defined MASSterclass technology on serum samples of said subjects and/or using immunohistochemistry with a Quiescin Q6-specific antibody on biopsies of said subjects, in order to confirm the overlap in tissue expression and blood circulation patterns of Quiescin Q6. Indeed, using immunohistochmistry, Quiescin Q6 expression has already been reported in the public domain: http://thorpelab.chem.udel.edu/index.php/qsox-ology. Amongst the reported tissues, also pancreatic, beta cells, show Quiescin Q6 expression. Interestingly, also insulinoma's, insulin producing tumours originating from beta cells show high expression of Quiescin Q6.

### Example 4: Quiescin Q6 levels in animal model of type 1 diabetes

Using a mouse or rat model of type 1 diabetes, the inventors can show that Quiescin Q6 expression is indeed linked to beta cell functioning and that it can be used to predict, diagnose, or for prognosis of beta cell dysfunction. In addition, the expression of Quiescin Q6 can be used to monitor beta cell disorder treatment. More specifically, increased Quiescin Q6 presence in a blood sample (whole blood, serum or plasma) of a subject is indicative of increased beta cell activity, e.g. reflected by increased insulin production. This can be due to beta cell function regeneration, reactivation, proliferation, maturation (or granulation) or the like.

Different animal models that mimic human type 1 diabetes have been described, with the NOD model being the best example.

Non-obese diabetic (NOD) mice exhibit a susceptibility to spontaneous development of T1 diabetes. The incidence of spontaneous diabetes in the NOD mouse is 60-80% in females and 20-30% in males. Auto-immune disease develops gradually and starts with the infiltration of immune cells into the pancreatic islets (insulitis). Onset of overt diabetes typically occurs around 12-14 weeks of age in female mice by which these mice have lost up to 70% of their β-cell activity. At different stages of the disease development Quiescin Q6 levels will be analyzed both in blood samples and in tissue sections of male and female mice that do and do not develop overt diabetes. Furthermore blood glucose and C-peptide levels will be monitored and β-cells will be imaged. Expression of Quiescin Q6 will thus be linked to β-cell activity and β-cell mass.

### Example 5: experiment in the T1DM patients

In T1D, a certain level of genetic predisposition has been reported among family members. Certain Diabetes Registers therefore collect samples at regular time points from siblings of known T1 D patients. Some siblings of those will likely develop T1 DM as well later on in life. Testing such serially obtained samples will allow to see what happens over time with certain markers including Quiescin Q6 during the evolution form pre-onset T1D, early onset T1D, pre-clinical T1D, to emerging T1 D with clear clinical signs.

In this experiment, we will investigate the level of Quiescin Q6 and other markers in samples around specific time points in the evolution. The following stages in T1 D disease progression will preferably be tested:
1. Siblings of T1 D patients with normal beta cell function/mass
2. The same subjects with emerging Auto-antibody titers to beta cells
3. The same subjects with more than 1, 2 or 3 Auto-antibodies to beta cells
4. The same subjects with initial beta cell destruction
5. The same subjects with reduced C-peptide levels
6. The same subjects with a disturbed Oral Glucose Tolerance Test
7. The same subjects with newly onset T1 D
8. The same subjects during the so-called honeymoon period
9. The same subjects with overt T1 D after said honeymoon period

## Claims

**1.** Use of Quiescin Q6, as a biomarker for measuring and/or monitoring beta cell mass and/or activity in a subject.

**2.** The use according to claim 1, wherein the beta cell activity in said subject is impaired or aberrant due to a condition selected from the group consisting of: pre-onset type 1 diabetes mellitus (T1D), pre-clinical T1D, early onset T1D, emerging T1D, pancreatitis such as: acute pancreatitis, alcohol induced acute pancreatitis, infectious acute pancreatitis, chronic pancreatitis, alcohol induced chronic pancreatitis, or biliar duct obstruction pancreatitis.

**3.** A method for the measurement and/or monitoring of beta cell mass and/or activity in a subject, comprising measuring the quantity of Quiescin Q6 in a blood sample from the subject.

**4.** The method according to claim 3, comprising:
(i) measuring the quantity of Quiescin Q6 in a blood sample from the subject;
(ii) comparing the quantity of said Quiescin Q6 measured in (i) with a reference value of the quantity of said Quiescin Q6, said reference value representing the beta cell activity in a reference sample;
(iii) finding a deviation or no deviation of the quantity of the Quiescin Q6 measured in (i) from the reference value; and
(iv) attributing said finding of deviation or no deviation to a particular diagnosis, prediction and/or prognosis of impaired or aberrant beta cell mass or activity in the subject.

**5.** The method according to claim 3 or 4, for determining whether a subject is or is not (such as, still is, or is no longer), or is to the same extent, in need of a therapeutic or prophylactic treatment of a beta cell related disease or disorder, selected from the group consisting of: pre-onset type 1 diabetes mellitus (T1D), pre-clinical T1D, early onset T1D, emerging T1D, or pancreatitis such as: acute pancreatitis, alcohol induced acute pancreatitis, infectious acute pancreatitis, chronic pancreatitis, alcohol induced chronic pancreatitis, or biliar duct obstruction pancreatitis.

**6.** The method according to any one claims 3 to 5, wherein the subject does not yet suffer from a clinically manifest beta cell related disease or disorder and whereby the probability, chance or risk that the subject will develop clinically manifest beta cell related disease or disorder is predicted.

**7.** The method according to any one of claims 3 to 6, performed at two or more successive time points, and attributing a finding of deviation or no deviation in Quiescin Q6 levels between said time points to a change in the beta cell activity and/or in disease progression.

**8.** The method according to any one claims 3 to 7, wherein the examination phase further comprises measuring the presence or absence and/or quantity of one or more other biomarkers useful for the measurement of beta cell mass or activity, such as insulin, pro-insulin, VMAT2, glucose, HbA1c, etc..

**9.** The method according to any one of claims 3 to 8, wherein the quantity of Quiescin Q6 is measured using (a) binding agent(s) capable of specifically binding to Quiescin Q6 and/or to fragments thereof, such as those selected from the group consisting of: antibodies or fragments thereof, aptamers, photoaptamers, proteins, peptides, polypeptides, peptidomimetics, DARPINS, or small molecules, or using an immunoassay technology, using a mass spectrometry analysis method, using a chromatography method, or using a combination of any of said methods.

**10.** Use of a kit comprising (i) means for measuring the quantity of Quiescin Q6 in a sample from the subject, and optionally and preferably (ii) a reference value of the quantity of Quiescin Q6 or means for establishing said reference value, wherein said reference value represents a known diagnosis, prediction and/or prognosis of a beta cell related disease or disorder, preferably wherein said means for measuring the quantity of Quiescin Q6 in a sample is a binding agent capable of specifically binding to said marker, such as those selected from the group consisting of: antibodies or fragments thereof, aptamers, photoaptamers, proteins, peptides, polypeptides, peptidomimetics, DARPINS, or small molecules, for measuring beta cell activity in a blood sample of a subject.

**11.** Use of a Quiescin Q6 protein, polypeptide, or peptide and/or a fragment thereof, preferably in a known quantity or concentration, for diagnosing a beta cell related disorder in a subject, by detecting the presence of autoantibodies to Quiescin Q6 in a sample of the subject.

**13.** Use of a testing device capable of measuring the quantity of Quiescin Q6 and/or fragments thereof in a sample from a subject comprising:
(i) means for measuring the quantity of Quiescin Q6 and/or fragments in said sample, and
(ii) means for visualising the quantity of Quiescin Q6 and/or fragments measured in the sample, for measuring beta cell activity in a sample of a subject.

**14.** The method according to any one of claims 3 to 9, for use in selection of patients that are at risk of progressing towards beta cell destruction/dysfunction, based on the levels of Quiescin Q6 in a blood sample of the subject.

**15.** The method according to any one of claims 3 to 9, for deciding on the timing of therapy to prevent, slow down or halt beta cell destruction/dysfunction, or to increase or restore beta cell activity, based on the levels of Quiescin Q6 in a blood sample of the subject.

**16.** The method according to any one of claims 3 to 9, for deciding on the type of therapy to prevent, slow down or halt beta cell destruction/dysfunction, or to increase or restore beta cell activity, based on the levels of Quiescin Q6 in a blood sample of the subject.

**17.** The method according to any one of claims 3 to 9, for use in designing or monitoring immunomodulating therapies, wherein the monitoring of the beta cell activity is used for modulating the treatment regimen or for selecting the most appropriate drugs or monoclonal antibodies for said immunomodulatory treatment.

**18.** The method according to any one of claims 3 to 9, for use in beta cell transplantation surveillance, wherein said method is used to measure the beta cell activity of the subject at certain time points after beta cell transplantation and wherein a change indicates that the grafted beta cells are changing their activity.

**19.** The method according to claim 18, for use in monitoring immunosuppression treatment, wherein increased immunosuppression therapy is advisable in case of reduction in beta cell activity, or wherein maintained or decreased immunosuppression therapy is advisable in case the beta cells activity are improving or in a steady state as compared to the pre-transplantation level.

**20.** The method according to any one of claims 3 to 9, for use in detecting or predicting the onset of insulin resistance or measuring the degree of IR in a subject.

**21.** The method or use according to any one of the previous claims, wherein said beta cell mass or activity reflects either one of: the capacity of insulin synthesis or secretion, the amount of insulin synthesis or secretion over a certain period, or the maximal amount of insulin secretion.

**22.** The method or use according to any one of the previous claims, wherein said beta cell mass or activity reflects either one of: the number or volume of beta cells, the number or volume of active beta cells, the number or volume of dormant beta cells, the rate of beta cell destruction, proliferation, regeneration or granulation in the beta cell tissue.

**23.** The method or use according to any one of the previous claims, wherein the severity of said impaired beta cell activity is measured in terms of either one of: the degree of autoimmune destruction of beta cells, the severity of Langerhans infiltration with white blood cells, the severity of auto-antigenecity, the correlation with the amount, titer or presence of auto-antibodies related to T1 D, such as GAD-65, pro-insulin, or IA2.

**24.** The use according to claim 1, the method according to claim 3 or 4, wherein the beta cell activity in said subject is impaired or aberrant due to a condition selected from the group consisting of: insulin resistance, exhaustion of beta cells, emerging or developing Type 2 Diabetes, pancreatic cancer or pancreatic tumour formation.
